# EUROPEAN PATENT SPECIFICATION

(11) **EP 4 069 206 B1**
(45) Date of publication and mention of the grant of the patent: **07.08.2024**
(21) Application number: 20829657.4
(22) Date of filing: 04.12.2020
(51) Int. Cl.: A61K 9/00, A61K 9/107, A61K 9/16, A61K 47/14, A61K 47/36, A61K 47/24, A61K 31/4402, A61P 3/10, A61P 21/00, A61K 33/24, A61P 3/02

(54) **SOLID FORM FORMULATION COMPRISING CHROMIUM OR COPPER OR A VITAMIN, COMPOSITIONS AND USES THEREOF**
FORMULIERUNG IN FESTER FORM UMFASSEND MIT CHROM ODER EIN KUPFER ODER EINEM VITAMIN, ZUSAMMENSETZUNGEN UND VERWENDUNGEN DAVON
FORMULATION SOUS FORME SOLIDE COMPRENANT DU CHROME OU DU CUIVRE OU UNE VITAMINE, COMPOSITIONS ET UTILISATIONS DE CELLE-CI

(30) Priority: 04.12.2019 IT 201900022989
(43) Date of publication of application: 12.10.2022
(60) Divisional application: 23202263.2
(73) Proprietor: Alesco S.r.l., 56122 Pisa (PI) (IT)
(72) Inventor: LACORTE, Andrea, 56122 Pisa (PI) (IT); TARANTINO, Germano, 56122 Pisa (PI) (IT); BRILLI, Elisa, 56122 Pisa (PI) (IT)
(74) Representative: Benedetto, Marco
(86) International application number: PCT/IB2020/061529
(87) International publication number: WO 2021/111407

(56) References cited:
- EVANS G W ET AL: "Chromium picolinate increases membrane fluidity and rate of insulin internalization", JOURNAL OF INORGANIC BIOCHEMISTRY, ELSEVIER INC, US, vol. 46, no. 4, 1 June 1992 (1992-06-01), pages 243 - 250, XP026629400, ISSN: 0162-0134, [retrieved on 19920601], DOI: 10.1016/0162-0134(92)80034-S
- SUSANA GÓMEZ-RAMÍREZ ET AL: "Sucrosomial Iron: A New Generation Iron for Improving Oral Supplementation", PHARMACEUTICALS, vol. 11, no. 4, 4 December 2018 (2018-12-04), CH, pages 97, XP055665686, ISSN: 1424-8247, DOI: 10.3390/ph11040097

## Description

The present invention relates to a solid form formulation of chromium, referred to as "sucrosomial chromium^{®}" (sucrosomial^{®} is a registered trademark on behalf of Pharmanutra S.p.A. and Alesco S.r.l.), comprising or, alternatively, consisting of at least one chromium salt or complex, at least one phospholipid, at least one first agent selected from a sucrester (alternatively called fatty acid carbohydrate ester), a cyclodextrin, a fatty acid and a chitosan derivative, and, optionally, a starch of plant origin.

Furthermore, the present invention relates to a composition, preferably in solid form, comprising said sucrosomial chromium^{®} and to said composition or formulation for use in the treatment of a chromium deficiency and in the treatment of a change in the carbohydrate metabolism and/or in the muscle energy metabolism.

Lastly, the present invention relates to a process for the preparation of said sucrosomial chromium^{®} and compositions thereof.

In addition, the present description relates to a solid form formulation based on copper (referred to as sucrosomial copper^{®}) comprising or, alternatively, consisting of: a copper salt or complex, at least one phospholipid, at least one sucrester, and, optionally, at least one starch of plant origin.

Furthermore, the present description relates to a composition, preferably in solid form, comprising said sucrosomial copper^{®} and additives and/or excipients and to the use of said composition or formulation based on copper in the treatment of a copper insufficiency or deficiency and of diseases and/or symptoms deriving from said insufficiency or deficiency.

Lastly, the present description relates to a process for the preparation of said formulation based on copper (sucrosomial copper^{®}) and compositions thereof.

In addition, the present description relates to a solid form formulation based on at least one vitamin (referred to as sucrosomial vitamin^{®}) comprising or, alternatively, consisting of: at least one vitamin (such as a vitamin A, a vitamins of group B (preferably B12), a vitamin C, a vitamin D (preferably D3) and/or a vitamin E), at least one phospholipid, at least one sucrester, and, optionally, at least one starch of plant origin.

Furthermore, the present description relates to a composition, preferably in solid form, comprising said at least one sucrosomial vitamin^{®} and additives and/or excipients and to the use of said composition or formulation in the treatment of an insufficiency or deficiency of said at least one vitamin and of diseases and/or symptoms deriving from said insufficiency or deficiency.

Lastly, the present description relates to a process for the preparation of said formulation based on said at least one vitamin (sucrosomial vitamin^{®}) and compositions thereof.

Chromium is an essential mineral for various vital functions in mammals. In particular, small amounts of chromium are necessary for the use of glucose by cells in response to insulin. Various studies have shown a correlation between chromium and glucose or insulin concentration in diabetic subjects, while results for non-diabetic subjects appear to be less significant.

Cellular energy metabolism comprises the set of processes that generate cellular energy (ATP) following the demolition of sugars (glycolysis), lipids and - in a very small percentage - proteins.

Chromium, in the Cr(lll) form thereof, is an essential element involved both in the metabolism of carbohydrates and in the metabolism of lipids, capable of enhancing the effects of insulin by lowering blood sugar and facilitating the entry of amino acids and lipids into cells. Considering that insulin has an anabolic action (enhances muscle glycogen synthesis), chromium may be capable of stimulating muscle amino acid incorporation and amplifying the anabolic action of insulin on the construction of new muscle tissue.

However, when chromium is administered to a human subject through the oral route (in short, *per os*), the gastrointestinal or intestinal absorption thereof and the blood bioavailability thereof may vary from subject to subject and/or not be particularly high and therefore cause significant differences in the response of subjects, with cases of poor efficacy. The low blood bioavailability of chromium has been estimated to be due to intestinal absorption ranging from 0.5% to 2% and urinary excretion ranging from 0.4% to 2.5%. Furthermore, the % gastrointestinal absorption of the chromium element varies depending on the chromium salt or complex administered.

Gary W. Evans et al. (Journal of Inorganic Biochemistry, 46, 1992) describes the effects of complexes containing chromium or zinc on the insulin internalization in cultured rat skeletal muscle cells. In particular, chromium picolinate showed the ability of increasing the insulin internalization in the cell cultures, accompanied by a marketed increase in the uptake of glucose and leucine. The article does not provide any hints relating the need of introducing a further component nor hypothesized a composition containing the chromium complex.

Copper too is an essential mineral for various vital functions in mammals. In particular, copper is involved in redox reactions and protein synthesis, for example for the production of enzymes. In the human body it plays a fundamental role in the formation of the biological respiratory catalyst cytochrome C oxidase.

Lastly, each of the vitamins that may be present in the formulations or compositions of the present description (such as vitamin A, of group B, C, D and E) plays fundamental roles for the metabolism and in the functioning and homeostasis mechanisms of cells and organs in subjects, in particular human subjects. Diseases or symptoms related to an insufficiency or deficiency of said vitamins may be, for example, cognitive problems, motor problems, decrease in immune defences and others as exemplified in the present description.

Therefore, there is a high need to provide novel formulations of chromium or copper or of at least one vitamin and compositions comprising said formulations of chromium and/or copper and/or vitamins which are highly absorbable at the gastrointestinal level and bioavailable at the blood level as well as highly effective in the supplementation of chromium and/or copper and/or vitamins, and/or in the treatment of an insufficiency or deficiency of chromium and/or copper and/or vitamins, for all categories of subjects.

Furthermore, there is a high need to provide novel formulations of chromium or copper or at least one vitamin and the compositions thereof which are highly absorbable at the gastrointestinal level and bioavailable at the blood level as well as highly effective in the treatment of changes in the carbohydrate metabolism and/or diseases and disorders related thereto, such as for example diabetes, hyperglycaemia, insulin resistance, high absorption of carbohydrates, deregulation of blood glucose level and/or metabolic syndrome, for all categories of subjects.

In addition, there is a high need to provide novel formulations of chromium or copper or at least one vitamin and the compositions thereof which are highly absorbable at the gastrointestinal level and bioavailable at the blood level, and effective in the treatment of changes in the muscle energy metabolism and/or disorders related thereto, such as for example decrease in muscle mass, decrease in muscle strength and decrease in physical resistance to muscle stress or to increase muscle energy metabolism and, therefore, increase muscle mass, increase muscle strength, increase physical resistance to muscle stress and reduce time to recover energy after a physical effort for all categories of subjects (both subjects with diseases or disorders and healthy subjects).

Lastly, the need is felt for said novel formulations of chromium or copper or at least one vitamin and the compositions thereof to be stable over time, well tolerated by all categories of subjects, cost-effective and easy to prepare.

Following extensive research and development activity, the Applicant addresses and solves the aforementioned needs by providing a novel chromium formulation, referred to as sucrosomial chromium^{®} and compositions comprising said sucrosomial chromium^{®}.

Furthermore, following extensive research and development activity, the Applicant addresses and solves the aforementioned needs by providing a novel copper formulation, referred to as a sucrosomial copper^{®} and compositions comprising said sucrosomial copper^{®} not encompassed by the wording of the claims.

Lastly, following extensive research and development activity, the Applicant addresses and solves the aforementioned needs by providing a novel formulation of at least one vitamin (such as vitamin A, of group B (*e.g.* B12), C, D (*e..g.* D3) and/or E) referred to as sucrosomial vitamin^{®} and compositions comprising said sucrosomial vitamin^{®} not encompassed by the wording of the claims.

An object of the present invention is to provide a novel formulation of chromium (sucrosomial chromium^{®}) and compositions thereof which are capable of increasing the effectiveness of a supplementation of chromium administered through the oral route, for example following an increase in gastrointestinal absorption and blood bioavailability of sucrosomial chromium^{®}.

In the context of the present invention, the terms gastrointestinal absorption and intestinal absorption are used interchangeably.

A further object of the present invention is to provide a novel chromium formulation (sucrosomial chromium^{®}) and compositions thereof which are effective and/or capable of increasing the effectiveness of a treatment for changes in the carbohydrate metabolism, such as diabetes, hyperglycaemia, insulin resistance, high absorption of carbohydrates, deregulation of blood glucose level and/or metabolic syndrome and/or which are effective and/or capable of increasing the effectiveness of a treatment for changes in the muscle energy metabolism. Said effectiveness and/or increased efficacy may for example be due to an increase in gastrointestinal absorption and blood bioavailability of sucrosomial chromium^{®}, and/or to an increase in the ability of cells to internalize blood glucose (blood glucose lowering action) and/or to an enhancement/improvement of muscle energy metabolism.

A good level of effectiveness and/or increased efficacy, even of a small extent, of the sucrosomial chromium^{®} according to the present invention with respect to chromium (or a chromium salt or complex) as such (i.e. chromium not formulated according to the invention) in the treatment of a chromium deficiency or changes in the carbohydrate metabolism or changes in the muscle energy metabolism or of other diseases/disorders defined in the present invention results in a decrease in the effective dose to be administered to a subject in need with respect to chromium (or chromium salt or complex) as such, and therefore it is also cost-effective.

Said good level or increased efficacy, even of a small extent, in the aforementioned treatments with sucrosomial chromium^{®} or the compositions thereof with respect to the treatments with chromium or a chromium salt or complex as such could be due to an increase in the blood bioavailability of chromium, in turn due to an increase in the intestinal or gastrointestinal absorption of sucrosomial chromium^{®} with respect to chromium (or chromium salt or complex) as such, when administered orally. However, said increased efficacy could be due to other mechanisms and reasons.

Said good level or increased efficacy in the treatment of the diseases or disorders defined in the present invention by using sucrosomial chromium^{®} or the compositions thereof subject of the present invention, with respect to the treatment with chromium (or a chromium salt or complex) as such, results in the attainment of a greater effect considering the same amount or concentration of chromium administered to a subject in need.

Similarly to what has been described for sucrosomial chromium^{®}, a sucrosomial vitamin^{®}, not encompassed by the wording of the claims, is more effective and/or capable of achieving equal effect at a lower dose with respect to said vitamin as such (i.e., vitamin not formulated according to the description), when administered to a subject in need or to a healthy subject (therapeutic use or non-therapeutic use). Such advantage of sucrosomial vitamin^{®} is assumed to be due to a greater intestinal or gastrointestinal absorption with respect to said vitamin as such.

Similarly to what has been described for sucrosomial chromium^{®}, a sucrosomial copper^{®}, not encompassed by the wording of the claims, is more effective and/or capable of attaining an equal effect at a lower dose with respect to copper as such (i.e. copper not formulated according to the description), when administered to a subject in need or to a healthy subject (therapeutic use or non-therapeutic use). Such advantage of sucrosomial copper^{®} is assumed to be due to a greater intestinal or gastrointestinal absorption with respect to said copper as such.

In addition, sucrosomial chromium^{®} and the compositions thereof, subject of the present invention, are stable over time from a chemical/physical and organoleptic point of view.

Lastly, sucrosomial chromium^{®} and the compositions thereof, subject of the present invention, do not have any significant side effects, are well tolerated and, therefore, they can be administered to all categories of subjects, including paediatric subjects, adolescents, pregnant or breastfeeding women and the elderly.

Furthermore, the process for the preparation of said sucrosomial chromium^{®} and the compositions thereof is easy to carry out or prepare and cost-effective in proportion to the treatment potential.

For example, the solid form formulation of chromium of the invention (sucrosomial chromium^{®}) can be easily processed to provide the compositions of the present invention, preferably in solid form for oral use.

These and other objects which will be apparent from the detailed description that follows, are achieved by the solid form formulation of chromium of the present invention (sucrosomial chromium^{®}) and/or by the solid form formulation of copper of the present description (sucrosomial copperr^{®}) and/or by the solid form formulation of a vitamin of the present description (sucrosomial vitamin^{®}) and the compositions thereof thanks to the technical characteristics reported in the present description and claimed in the attached claims.

### DESCRIPTION OF THE FIGURES

Figure 1: graphical representation of the glucose assay data of the 2 compositions under analysis.
Figure 2: graphical representation of the GLU-4 assay data of the 2 compositions under analysis.
Figure 3: graphical representation of ATP assay data of the 2 compositions under analysis.

### SUMMARY OF THE INVENTION

An object of the present invention is to provide a solid form formulation of chromium comprising or, alternatively, consisting of chromium (a chromium salt or complex), a phospholipid, a sucrester and, optionally, a plant starch (in short, chromium formulation or formulation of the present invention).

An object of the present description is to provide a solid form formulation of copper comprising or, alternatively, consisting of copper (a copper salt or complex), a phospholipid, a sucrester and, optionally, a plant starch (in short, copper formulation or formulation of the present descriptopn).

An object of the present description is to provide a solid form formulation of at least one vitamin comprising or, alternatively, consisting of at least one vitamin, a phospholipid, a sucrester and, optionally, a plant starch (in short, formulation of at least one vitamin or formulation of the present description).

An object of the present invention is to provide compositions, preferably in solid form, comprising at least one formulation of the present invention of chromium and pharmaceutical or food grade additives and/or excipients (in short, compositions of the present invention).

An object of the present invention is to provide said formulations of chromium or compositions of the present invention for use as medicament.

An object of the present invention is to provide said formulations of chromium or compositions of the present invention for use in a method for the treatment of a deficiency or insufficiency of chromium, or for use in a method for the treatment of disorders of the carbohydrate or muscle energy metabolism.

An object of the present invention is to provide a non-therapeutic use (or cosmetic use) of said formulations of chromium or compositions of the present invention for the supplementation of chromium to a healthy subject in order to increase the physical and/or mental performance thereof or for cosmetic purposes.

### DETAILED DESCRIPTION OF THE INVENTION

Forming an object of the present description is a solid form formulation of chromium referred to as sucrosomial chromium^{®} (in short, chromium formulation of the invention) comprising or, alternatively, consisting of: (a) at least one chromium salt or chromium complex, (b) at least one phospholipid, preferably a phosphoglyceride (or glycerophospholipids), (c) at least one first agent selected from the group comprising or, alternatively, consisting of: (c-i) at least one fatty acid carbohydrate ester (alternatively referred to as sucrester); (c-ii) at least one cyclodextrin, preferably an α-cyclodextrin; (c-iii) at least one fatty acid having a number of carbon atoms comprised in the range from C6 to C18, preferably from C12 to C18; (c-iv) at least one chitosan derivative, preferably carboxymethylchitosan; and mixtures thereof; and, optionally, (d) at least one starch of plant origin, preferably a gelatinised or pre-gelatinised starch of plant origin, even more preferably a rice starch.

The solid form formulation of chromium of the present invention comprises or, alternatively, consists of: (a) a chromium salt or chromium complex, (b) a phospholipid, preferably a phosphoglyceride (or glycerophospholipids), (c-i) a sucrester, and, optionally, (d) at least one starch of plant origin, preferably a gelatinised or pre-gelatinised starch of plant origin, more preferably a rice starch or pre-gelatinised rice starch.

A preferred example of said solid form formulation of chromium comprises or, alternatively, consists of: (a) a chromium salt or chromium complex, (b) a lecithin (c-i) a sucrester, and, optionally, (d) at least one starch of plant origin, preferably a gelatinised or pre-gelatinised starch of plant origin, more preferably a rice starch or pre-gelatinised rice starch.

In a preferred embodiment of the present invention, said solid form formulation of chromium (sucrosomial chromium^{®}) comprises or, alternatively, consists of: (a) at least one chromium salt or chromium complex, (b) at least one phospholipid, preferably a phosphoglyceride, (c) at least one first agent comprising or, alternatively, consisting of (c-i) a fatty acid carbohydrate ester (alternatively referred to as sucrester) and (d) at least one starch of plant origin, preferably a gelatinised or pre-gelatinised starch of plant origin, even more preferably a rice starch or pre-gelatinised rice starch.

In the context of the present invention, the terms "fatty acid carbohydrate ester", "sucrester" and "sucrose fatty acid ester", both used in singular and plural form, are synonyms and they identify the same compound.

In an embodiment of the present invention said at least one chromium salt or chromium complex (a), comprised in the chromium formulation of the invention (sucrosomial chromium^{®}) together with (b), (c-i) and, optionally, (d), is selected gto the group A comprising or, alternatively, consisting of: chromium picolinate, chromium histidinate, chromium tris-histidinate or, chromium polyhistidinate, chromium nicotinate, chromium polynicotinate, chromium dinicocysteinate, chromium dinicotinate tryptophan, chromium dinicotinate tyrosine, chromium dinicotinate hydroxycitrate, chromium dinicotinate cinnamate, chromium dinicotinate gallate, chromium dinicotinate 5 hydroxytryptophan, chromium dinicotinate aspartate, chromium dinicotinate glutamate, chromium dinicotinate arginate; chromium tris-tryptophan, chromium nicotinate glycinate, chromium phenylalanine, chromium triphenylalanine, chromium tris-tyrosine, chromium tris-hydroxycitrate, chromium tris-(5-hydroxytryptophan), chromium tris-cinnamate, chromium tris-gallate, chromium acetate, chromium sulphate, chromium (III) chloride, chromium yeast; preferably said (a) chromium salt or complex is chromium (iii) picolinate or chromium (iii) nicotinate or polinicotinate or chromium (III) chloride or a mixture thereof; more preferably said (a) chromium salt or complex is chromium (iii) picolinate.

Chromium picolinate or chromium (iii) picolinate (IUPAC name chromium (III) picolinate, molecular formula C₁₈H₁₂N₃O₆Cr, molecular weight 418.33, identified for example by CAS No. 14639-25-9) is a compound for coordinating the chromium cation having a +3 valence with three units of picolinic acid, each having a -1 valence. In the context of the present invention, the terms chromium picolinate, chromium (III) picolinate and chromium tripicolinate are synonyms.

Chromium nicotinate or chromium (III) nicotinate contains three units of nicotinic acid per chromium cation with a +3 valence (chromium (iii) trinicotinate). In the context of the present invention, the terms chromium nicotinate, chromium (iii) nicotinate and chromium trinicotinate are synonyms.

Chromium polycotinate is a mixture of trinicotinate and dinicotinate, with the dominant trinicotinate.

Chromium chloride or chromium trichloride or chromium chloride (iii) is the chemical compound of formula CrCl₃. It exists in different forms depending on the degree of hydration, for example chromium trichloride hexahydrate. Advantageously, the chromium (III) chloride used in the present invention is Cr(lll) chloride hexahydrate.

In the context of the present invention, the terms chromium chloride or chromium trichloride or chromium (III) chloride are synonyms.

In an embodiment of the present invention, the solid form formulation of chromium (sucrosomial chromium^{®}) comprises or, alternatively, consists of: (a) chromium picolinate; (b) at least one phospholipid, preferably a phosphoglyceride, more preferably a phosphatidylcholine or lecithin, even more preferably a sunflower lecithin; (c) at least one first agent selected from: (c-i) at least one sucrester, and, optionally, (d) at least one starch of plant origin, preferably a gelatinised or pre-gelatinised starch of plant origin, even more preferably a rice starch or pre-gelatinised rice starch.

In a preferred embodiment of the present invention, said solid form formulation of chromium (sucrosomial chromium^{®}) comprises or, alternatively, consists of: (a) chromium picolinate; (b) at least one phospholipid, preferably a phosphoglyceride; (c-i) at least one sucrester; and, optionally, (d) at least one starch of plant origin, preferably a gelatinised or pre-gelatinised starch of plant origin, even more preferably a rice starch or pre-gelatinised rice starch.

In a more preferred embodiment of the present invention, said solid form formulation of chromium (sucrosomial chromium^{®}) comprises or, alternatively, consists of: (a) chromium picolinate; (b) at least one phosphatidylcholine or lecithin (for example sunflower, corn and/or soy lecithin), preferably sunflower lecithin; (c-i) at least one sucrester; and, optionally, (d) at least one gelatinised or pre-gelatinised starch of plant origin, preferably rice starch or pre-gelatinised rice starch.

For example, said solid form formulation of chromium (sucrosomial chromium^{®}) comprises or, alternatively, consists of: (a) chromium picolinate, (b) a sunflower lecithin, preferably non-hydrolised or non-enzymatically hydrolised, (c-i) a sucrester, and, optionally, (d) pre-gelatinised rice starch.

For example, said solid form formulation of chromium (sucrosomial chromium^{®}) comprises or, alternatively, consists of: (a) chromium picolinate, (b) a sunflower lecithin, preferably non-hydrolised or non-enzymatically hydrolised, (c-i) a sucrester, and (d) pre-gelatinised rice starch.

In an embodiment of the present invention, the solid form formulation of chromium (sucrosomial chromium^{®}) comprises or, alternatively, consists of: (a) chromium nicotinate or polynicotinate; (b) at least one phospholipid, preferably a phosphoglyceride, more preferably a phosphatidylcholine or lecithin, even more preferably sunflower lecithin; (c) at least one first agent selected from: (c-i) at least one sucrester; and, optionally, (d) at least one starch of plant origin, preferably a gelatinised or pre-gelatinised starch of plant origin, even more preferably a rice starch.

In an embodiment of the present invention, the solid form formulation of chromium (sucrosomial chromium^{®}) comprises or, alternatively, consists of: (a) chromium chloride; (b) at least one phospholipid, preferably a phosphoglyceride, more preferably a phosphatidylcholine or lecithin, even more preferably sunflower lecithin; (c) at least one first agent selected from: (c-i) at least one sucrester; and, optionally, (d) at least one starch of plant origin, preferably a gelatinised or pre-gelatinised starch of plant origin, even more preferably a rice starch.

Forming an object of the present description is a solid form formulation of copper of copper (in short, copper formulation of the description or sucrosomial copper^{®}) comprising or, alternatively, consisting of;
(s) copper, for example in the form of a copper salt or complex, preferably copper (II), more preferably copper gluconate (Cu(2+)) or copper sulphate (Cu(2+);
(b) at least one phospholipid, preferably a phosphoglyceride, more preferably a phosphatidylcholine or lecithin;
(c-i) at least one sucrester or fatty acid carbohydrate ester;
and, optionally, (d) at least one starch of plant origin, preferably gelatinised or pre-gelatinised plant starch, preferably rice starch.

Said solid form formulation of copper, not encompassed by the wording of the claims, may comprise or, alternatively, consist of: (a) a copper salt or complex (e.g. Cu(II)), (b) a sunflower, corn or soy lecithin (e.g. sunflower (E322)), (c-i) a sucrester (e.g. E473), and, optionally, (d) a starch of plant origin, preferably a gelatinised or pre-gelatinised rice starch.

An example of said solid form formulation of copper, not encompassed by the wording of the claims, comprises or, alternatively, consists of: (a) copper gluconate, (b) a phospholipid, preferably a phosphoglyceride (or glycerophospholipids), (c-i) a sucrester, and, optionally, (d) a starch of plant origin, preferably gelatinised or pre-gelatinised, more preferably a rice starch or pre-gelatinised rice starch.

A preferred example of said solid form formulation of copper, not encompassed by the wording of the claims, comprises or, alternatively, consists of: (a) copper gluconate, (b) a lecithin, preferably sunflower, corn and/or soy lecithin, (c-i) a sucrester, and, optionally, (d) a starch of plant origin, preferably gelatinised or pre-gelatinised, more preferably a rice starch or pre-gelatinised rice starch.

A further preferred example of said solid form formulation of copper, not encompassed by the wording of the claims, comprises or, alternatively, consists of: (a) copper gluconate, (b) a sunflower lecithin (e.g. sunflower (E322)), (c-i) a sucrester (e.g. E473), and, optionally, (d) a starch of plant origin, preferably a rice starch or pre-gelatinised rice starch.

A further preferred example of said solid form formulation of copper, not encompassed by the wording of the claims, comprises or, alternatively, consists of: (a) copper gluconate, (b) a sunflower lecithin (e.g. sunflower (E322)), (c-i) a sucrester (e.g. E473), and (d) a pre-gelatinised rice starch.

Forming an object of the present description is a solid form formulation of at least one vitamin (in short, formulation of a vitamin of the description or formulation of the description or, alternatively, sucrosomial vitamin^{®}) comprising or, alternatively, consisting of:
(a) at least one vitamin, wherein said vitamin is selected from the group comprising or, alternatively, consisting of: (a-I) at least one vitamin of group B (for example, B12, B9, B6, B3 or B2), preferably vitamin B12, (a-II) a vitamin C, (a-III) a vitamin D, preferably D3, (a-IV) a vitamin E, (a-V) a vitamin A and mixtures thereof;
(b) at least one phospholipid, preferably a phosphoglyceride, more preferably a phosphatidylcholine or lecithin;
(c-i) at least one sucrester or a fatty acid carbohydrate ester;
and, optionally, (d) at least one starch of plant origin, preferably gelatinised or pre-gelatinised, preferably rice starch or pre-gelatinised rice starch.

The solid form formulation of vitamin B12 (sucrosomial vitamin B12^{®}), not encompassed by the wording of the claims, comprises or, alternatively, consists of: (a) vitamin B12, (b) at least one phospholipid, preferably a phosphatidylcholine or lecithin; (c-i) a sucrester; and, optionally, (d) a starch of plant origin, preferably gelatinised or pre-gelatinised.

The solid form formulation of vitamin C (sucrosomial vitamin C^{®}), not encompassed by the wording of the claims, comprises or, alternatively, consists of: (a) vitamin C, (b) at least one phospholipid, preferably a phosphatidylcholine or lecithin; (c-i) a sucrester; and, optionally, (d) a starch of plant origin, preferably gelatinised or pre-gelatinised.

The solid form formulation of vitamin D (sucrosomial vitamin D ^{®}), not encompassed by the wording of the claims, comprises or, alternatively, consists of: (a) vitamin D, (b) at least one phospholipid, preferably a phosphatidylcholine or lecithin; (c-i) a sucrester; and, optionally, (d) a starch of plant origin, preferably gelatinised or pre-gelatinised.

The solid form formulation of vitamin E (sucrosomial vitamin E^{®}), not encompassed by the wording of the claims, comprises or, alternatively, consists of: (a) vitamin E, (b) at least one phospholipid, preferably a phosphatidylcholine or lecithin; (c-i) a sucrester; and, optionally, (d) a starch of plant origin, preferably gelatinised or pre-gelatinised.

The solid form formulation of vitamin A (sucrosomial vitamin A^{®}), not encompassed by the wording of the claims, comprises or, alternatively, consists of: (a) vitamin A, (b) at least one phospholipid, preferably a phosphatidylcholine or lecithin; (c-i) a sucrester; and, optionally, (d) a starch of plant origin, preferably gelatinised or pre-gelatinised.

The solid form formulation a of vitamin B (sucrosomial vitamin B^{®}), not encompassed by the wording of the claims, comprises or, alternatively, consists of: (a) a vitamin B (for example B3, B6 or B9), (b) at least one phospholipid, preferably a phosphatidylcholine or lecithin; (c-i) a sucrester; and, optionally, (d) a starch of plant origin, preferably gelatinised or pre-gelatinised.

An example of said solid form formulation of at least one vitamin, not encompassed by the wording of the claims, comprises or, alternatively, consists of:
(a) a vitamin selected from the group comprising or, alternatively, consisting of: (a-I) vitamin B12, (a-II) vitamin C, (a-III) vitamin D3, (a-IV) vitamin E;
(b) a lecithin, preferably a sunflower, corn or soy lecithin (preferably sunflower (E322));
(c-i) a sucrester (e.g. sucrester E473);
and, optionally, (d) a gelatinised or pre-gelatinised starch of plant origin, preferably pre-gelatinised rice starch.

A preferred example of said solid form formulation based on vitamin, not encompassed by the wording of the claims, comprises or, alternatively, consists of:
(a) a vitamin selected from the group comprising or, alternatively, consisting of: (a-I) vitamin B12, (a-II) vitamin C, (a-III) vitamin D3, (a-IV) vitamin E;
(b) a lecithin, preferably a sunflower, corn or soy lecithin (preferably sunflower (E322));
(c-i) a sucrester (e.g. sucrester E473); and
(d) a gelatinised or pre-gelatinised starch of plant origin, preferably pre-gelatinised rice starch.

A further preferred example of said solid form formulation based on vitamin, not encompassed by the wording of the claims, comprises or, alternatively, consists of:
(a) a vitamin selected from the group comprising or, alternatively, consisting of: (a-I) vitamin B12, (a-II) vitamin C, (a-III) vitamin D3, (a-IV) vitamin E;
(b) a sunflower lecithin (e.g. E322);
(c-i) a sucrester (e.g. E473); and, optionally,
(d) a pre-gelatinised rice starch.

Phospholipids are phosphate-containing lipids. The molecules of this class of organic compounds have a water-soluble polar head (i.e., water-soluble and insoluble in apolar solvents) based on phosphate and a hydrophobic non-water-soluble apolar tail (i.e., water-insoluble and soluble in apolar solvents), for this reason they are referred to as amphipathic molecules.

Phosphoglycerides (also called glycerophospholipids) represent the most important class of phospholipids. Glycerophospholipids (or phosphoglycerides) are all derived from sn-glycerol-3-phosphate, in which glycerol (CH₂OH-CHOH-CH₂OH) is esterified in position 3 with orthophosphoric acid (H₃PO₄). In glycerophospholipids, glycerol is esterified in position 2 with a fatty acid, while in position 1 different classes of compounds can be bound; since carbon 2 is asymmetric, there are two possible stereoisomers: L and D. In nature glycerophospholipids all belong to the L series.

Depending on the nature of the molecule that binds to the position 1 of glycerol, three subclasses of phosphoglycerides can be distinguished: 1,2-di-acyl-phospholipids, 1-alkyl-2-acyl-phospholipids, 1-alkenyl-2-acyl-phospholipids.

Diacyl-phospholipids (phosphoglycerides) derive from the structure of triglycerides, where a fatty acid is replaced by a phosphate group which gives the molecule a negative charge and therefore polarity; this molecule has the generic name phosphatide. A more complex organic molecule - generally serine, choline, ethanolamine, inositol or a single hydrogen atom - is bound to the phosphate group - through an ester bond - obtaining a phospholipid called, respectively, phosphatidylserine, phosphatidylcholine, phosphatidylethanolamine, phosphatidylinositol or phosphatidic acid.

Diacyl-phospholipids are characterised by a water-soluble polar head, which dissolves well in water, while the two saturated fatty acids represent the two apolar tails, which are not water-soluble but lipophilic.

Advantageously, the (b) phospholipids of the present invention, comprised in the chromium formulation of the invention (sucrosomial chromium^{®}) or in the copper formulation of the description (sucrosomial copper^{®}) or in the vitamin formulation of the description (sucrosomial vitamin^{®}) (together with (a), (c) such as (c-i) and, optionally, (d)), are phosphoglycerides selected from 1,2-diacyl-phospholipids, 1-alkyl-2-acyl-phospholipids, 1-alkenyl-2-acyl-phospholipids, preferably diacyl-phospholipids.

In an embodiment of the present invention, said at least one phospholipid (b), comprised in the chromium formulation of the invention (sucrosomial chromium^{®}) or in the copper formulation of the description (sucrosomial copper^{®}) or in the vitamin formulation of the description (sucrosomial vitamin^{®}) (together with (a), (c) such as (c-i) and, optionally, (d)), is a phosphoglyceride, preferably a diacyl-phospholipid, more preferably selected from the group B comprising or, alternatively, consisting of: phosphatidylcholine or lecithin, phosphatidylserine, phosphatidylethanolamine, phosphatidylinositol, phosphatidic acid and the mixtures thereof; preferably phosphatidylcholineor lecithin (E322); even more preferably phosphatidylcholine or sunflower, corn or soy lecithin (E322); even more preferably allergen free.

In a preferred embodiment of the present invention, the solid form formulation of chromium (sucrosomial chromium^{®}) comprises or, alternatively, consists of: (a) chromium picolinate; (b) at least one phospholipid selected from the group B comprising or, alternatively, consisting of: phosphatidylcholine or lecithin, phosphatidylserine, phosphatidylethanolamine, phosphatidylinositol, phosphatidic acid and mixtures thereof; (c) at least one first agent selected from: (c-i) at least one sucrester, and, optionally, (d) at least one starch of plant origin, preferably a gelatinised or pre-gelatinised starch of plant origin, even more preferably a rice starch.

In a more preferred embodiment of the present invention, the solid form formulation of chromium (sucrosomial chromium^{®}) comprises or, alternatively, consists of: (a) chromium picolinate; (b) at least one phospholipid selected from group B comprising or, alternatively, consisting of: phosphatidylcholine or lecithin, phosphatidylserine, phosphatidylethanolamine, phosphatidylinositol, phosphatidic acid and mixtures thereof; (c-i) at least one sucrester, and, optionally, (d) at least one starch of plant origin, preferably a gelatinised or pre-gelatinised starch of plant origin, even more preferably a rice starch.

According to an example not encompassed by the wording of the claims, the solid form formulation of copper (sucrosomial copper^{®}) comprises or, alternatively, consists of: (a) copper, preferably copper gluconate; (b) at least one phospholipid selected from the group B comprising or, alternatively, consisting of: phosphatidylcholine or lecithin, phosphatidylserine, phosphatidylethanolamine, phosphatidylinositol, phosphatidic acid and mixtures thereof; (c-i) a sucrester, and, optionally, (d) a starch of plant origin preferably pre-gelatinised rice starch.

According to an example not encompassed by the wording of the claims, the solid form formulation of a vitamin (sucrosomial vitamin^{®}) comprises or, alternatively, consists of: (a) a vitamin selected from vitamin B12, C, D3 and E; (b) at least one phospholipid selected from the group B comprising or, alternatively, consisting of: phosphatidylcholine or lecithin, phosphatidylserine, phosphatidylethanolamine, phosphatidylinositol, phosphatidic acid and mixtures thereof; (c-i) a sucrester, and, optionally, (d) a starch of plant origin, preferably pre-gelatinised rice starch.

The term lecithin is used to indicate a class of chemical compounds present in animal and plant tissues (particularly in the egg yolk).

From a chemical point of view, a lecithin is a phosphatidylcholine ((R)-1-Oleoyl-2-palmitoyl-phosphatidylcholine) or a lecithin comprising - as primary component - one or more phospholipids, preferably phosphatidylcholine.

A phosphatidylcholine is a phosphoglyceride in which phosphatidic acid is esterified with choline. Phosphatidic acids represent the simplest phosphoglycerides, formally produced by the esterification of glycerol in position 1 and 2 with fatty acids and in position 3 with orthophosphoric acid.

Lecithin is a natural emulsifier due to its chemical/physical properties, and it has a secondary antioxidant function, given that it is rich in natural antioxidant substances.

In an embodiment of the present description, the (b) phosphatidylcholine or lecithin, comprised in the chromium formulation of the invention (sucrosomial chromium^{®}) or in the copper formulation of the description (sucrosomial copper^{®}) or in the vitamin formulation of the description (sucrosomial vitamin^{®}) (together with (a), (c) such as (c-i) and, optionally, (d)), is a phosphatidylcholine or lecithin in solid form, such as for example in the form of powder or granules, preferably solid lecithin E322, more preferably a solid lecithin E322 selected from sunflower lecithin, corn lecithin and soy lecithin; even more preferably sunflower lecithin (E322), for example allergen free solid sunflower lecithin E322 (e.g. powdered or granular). The term "allergen free" means that it does not have any allergen residues. The term "E322" indicates that lecithin is a food additive (emulsifier) permitted by European legislation and regulated by the Italian Ministerial Decree No. D.M. 1996.

Directive 2008/84/EC of 27 August 2008 (published in the Official Journal of the European Union No L253) lays down the purity criteria which a lecithin must meet in order to be considered to meet food quality standards (lecithin E322): insoluble in acetone (basically the active part of lecithin): 60% min.; moisture: 2% max.; acidity number: 35 max.; peroxides number: 10 max.; insoluble in toluene (the impurities basically): 0.3% max.

Should said (b) phosphatidylcholine or lecithin be a powdered or granular lecithin, lecithin may for example have a % by weight water content comprised in a range from 1.5% to 4.5% with respect to the weight of lecithin, preferably from 2% to 4%, even more preferably from 2.5% to 3.5%.

When said (b) phosphatidylcholine or lecithin is a sunflower lecithin E322, preferably powdered or granular, lecithin may have, for example, a % by weight glucose content comprised in the range from 20% to 60% with respect to the weight of lecithin, preferably from 30% to 50%, for example about 45%. A (b) sunflower lecithin E322, preferably in form of powder or granules, that can be used in the context of the present invention may have the following composition at a % by weight (chemical/physical analysis): sunflower lecithin from 40% to 50%, carbohydrates from 40% to 50% (for example about 42%), proteins from 6% to 10%, ashes from 3% to 8%, moisture from 2% to 5% and another flowing agent from 0.5% to 1.5%.

In an embodiment of the present description, said (c-i) sucrester (or fatty acid carbohydrate ester), comprised in the chromium formulation of the invention (sucrosomial chromium^{®}) or in the copper formulation of the description (sucrosomial copper^{®}) or in the vitamin formulation of the description (sucrosomial vitamin^{®}) (together with (a), (b) and, optionally, (d)), is a sucrester E473, preferably a sucrester E473 comprising at least 50% by weight, preferably from 70% by weight to 90% by weight, with respect to the total weight of the sucrester, of monoesters obtained by esterification of sucrose with one or more fatty acids of plant origin, preferably stearic acid and/or palmitic acid. The abbreviation "E473" is used to indicate that sucresters or sucrose fatty acid esters are food additives (emulsifiers) permitted by European legislation and regulated by the Italian Ministerial Decree D.M. 1996.

"Sucresters" are generally obtained from the esterification of the fatty acids or from the trans-esterification of fatty acids methyl esters with carbohydrates (also called saccharides). Generally, the carbohydrates used are sucrose (monosaccharide) and polysaccharides, so sucresters are also referred to as "sucrose ester fatty acids". The chemical/physical properties of these compounds depend on the number and the type of esterified fatty acids. They are essentially emulsifiers and they are added to the compositions so as to determine a greater stabilisation of an aqueous phase with a fatty phase.

For example, a (c-i) sucrester (E473) that can be used in the context of the present invention may be a sucrester having an HLB (hydrophilic-lipophilic balance) comprised in the range from 14 to 18, preferably an HLB value of about 15 or 16.

A (c-i) sucrester (E473) that can be used in the context of the present invention may have the following composition by weight: total ester content of at least 90%, of which at least 70% by weight, with respect to the total weight of the sucrester, of monoesters obtained through the esterification of sucrose with one or more fatty acids of plant origin, preferably stearic acid and/or palmitic acid; free fatty acid content (such as oleic acid) not exceeding 3 %; free sucrose content not exceeding 2 %; moisture not exceeding 4 %; acid value not exceeding 5. An example of (c-i) commercial sucrester that can be used in the context of the present invention is: sucrose esters SP70 produced by Chimab S.p.A -Italia.

In an embodiment of the present description, said (c-i) sucrester, comprised in the chromium formulation of the invention (sucrosomial chromium^{®}) or in the copper formulation of the description (sucrosomial copper^{®}) or in the vitamin formulation of the description (sucrosomial vitamin^{®}) (together with (a), (b) and, optionally, (d)), does not comprise or, alternatively, it does not consist of a polyglycerol fatty acid ester.

In an embodiment of the present description, said (b) lecithin, comprised in the chromium formulation of the invention (sucrosomial chromium^{®}) or in the copper formulation of the description (sucrosomial copper^{®}) or in the vitamin formulation of the description (sucrosomial vitamin^{®}) (together with (a), (c) such as (c-i) and, optionally, (d)) does not comprise or, alternatively, it does not consist of a decomposed or hydrolysed lecithin and it does not comprise or, alternatively, it does not consist of an enzymatically decomposed or hydrolysed lecithin (i.e., enzymatically decomposed or hydrolysed).

In an embodiment of the present description, the chromium formulation of the invention (sucrosomial chromium^{®}) or the copper formulation of the description (sucrosomial copper^{®}) or the vitamin formulation of the description (sucrosomial vitamin^{®}) further comprises, together with (a), (b) and (c) such as (c-i) a (d) starch of plant origin, preferably gelatinised or pre-gelatinised; preferably, said (d) starch of plant origin is selected from rice starch and/or corn starch; preferably, said (d) starch of plant origin is rice starch (Oryza *sativa*) or native rice starch, preferably gelatinised or pre-gelatinised; more preferably, said (d) starch of plant origin is pre-gelatinised rice starch.

A pre-gelatinised rice starch (d) that can be used in the context of the present invention may for example have the following chemical-physical characteristics: humidity not exceeding 7%; protein content not exceeding 1%; ash content not exceeding 1%; pH (10% solution) comprised from 5.5 and 7.5, density 0.40-0.48 g/cm3; minimum starch content at 97% and fat content not exceeding 0.1 %. An example of commercial pre-gelatinised rice starch is AX-FG-P manufactured by Reire Srl -Italia.

In a preferred embodiment of the present invention, the solid form formulation of chromium of the invention (sucrosomial chromium^{®}) comprises or, alternatively, consists of: (a) at least one chromium salt or chromium complex, preferably chromium picolinate (b) at least one phospholipid, preferably a phosphoglyceride, (c-i) at least one sucrester and (d) at least one starch of plant origin.

In a more preferred embodiment of the present invention, the solid form formulation of chromium of the invention (sucrosomial chromium^{®}) comprises or, alternatively, consists of: (a) at least one chromium salt or chromium complex selected from the group A as defined above, preferably chromium picolinate; (b) a phosphatidylcholine or lecithin (for example sunflower, corn and/or soy lecithin), preferably a solid lecithin (E322), more preferably solid sunflower lecithin (E322), even more preferably allergen free solid sunflower lecithin (E322); (c-i) a sucrester (E473), preferably sucrester (E473) comprising at least 70% by weight, with respect to the total weight of the sucrester, of monoesters obtained by esterification of sucrose with one or more fatty acids of plant origin, preferably stearic acid and/or palmitic acid; and (d) a pre-gelatinised starch of plant origin, preferably pre-gelatinised rice starch.

In an even more preferred embodiment of the present invention, the solid form formulation of chromium of the invention (sucrosomial chromium^{®}) comprises or, alternatively, consists of: (a) chromium picolinate; (b) an allergen free phosphatidylcholine or solid sunflower lecithin (E322); (c-i) a sucrester (E473) comprising at least 70% by weight, with respect to the total weight of the sucrester, of monoesters obtained by esterification of sucrose with one or more fatty acids of plant origin, preferably stearic acid and/or palmitic acid; and (d) a pre-gelatinised rice starch.

According to an example, the solid form formulation of copper of the description (sucrosomial copper^{®}) comprises or, alternatively, consists of: (a) copper; preferably copper gluconate; (b) phosphatidylcholine or lecithin (for example sunflower, corn and/or soy lecithin), preferably sunflower lecithin (E322); (c-i) a sucrester (E473), comprising at least 70% by weight, with respect to the total weight of the sucrester, of monoesters obtained by esterification of sucrose with one or more fatty acids of plant origin, preferably stearic acid and/or palmitic acid; and, optionally, and (d) a pre-gelatinised starch of plant origin, preferably pre-gelatinised rice starch.

According to an example, the solid form formulation of copper of the description (sucrosomial copper^{®}) comprises or, alternatively, consists of: (a) copper gluconate; (b) an allergen free phosphatidylcholine or solid sunflower lecithin (E322); (c-i) a sucrester (E473) comprising at least 70% by weight, with respect to the total weight of the sucrester, of monoesters obtained by esterification of sucrose with one or more fatty acids of plant origin, preferably stearic acid and/or palmitic acid; and (d) a pre-gelatinised rice starch.

According to an example, the solid form formulation of a vitamin of the description (sucrosomial vitamin^{®}) comprises or, alternatively, consists of: (a) at least one vitamin selected from the group comprising or, alternatively, consisting of: (a-I) vitamin B12, (a-II) vitamin C, (a-III) vitamin D3, and (a-IV) vitamin E; (b) a phosphatidylcholine or lecithin (for example sunflower, corn and/or soy lecithin), preferably a sunflower lecithin (E322); (c-i) a sucrester (E473) comprising at least 70% by weight, with respect to the total weight of the sucrester, of monoesters obtained by esterification of sucrose with one or more fatty acids of plant origin, preferably stearic acid and/or palmitic acid; and, optionally, (d) a pre-gelatinised starch of plant origin, preferably pre-gelatinised rice starch.

According to an example, the solid form formulation of a vitamin of the description (sucrosomial vitamin^{®}) comprises or, alternatively, consists of: (a) at least one vitamin selected from the group comprising or, alternatively, consisting of: (a-I) vitamin B12, (a-II) vitamin C, (a-III) vitamin D3 and (a-IV) vitamin E; (b) an allergen free phosphatidylcholine or solid sunflower lecithin (E322); (c-i) a sucrester (E473) comprising at least 70% by weight, with respect to the total weight of the sucrester, of monoesters obtained by esterification of sucrose with one or more fatty acids of plant origin, preferably stearic acid and/or palmitic acid; and (d) a pre-gelatinised rice starch.

In an alternative example, not encompassed by the wording of the claims, of the present description, the chromium formulation of the description (sucrosomial chromium^{®}) comprises, together with (a), (b) and, optionally, (d) according to any one of the described examples, said at least one first agent (c) comprising or, alternatively, consisting of (c-ii) at least one cyclodextrin, preferably an α-cyclodextrin.

In an alternative example, not encompassed by the wording of the claims, of the present description, the chromium formulation of the description (sucrosomial chromium^{®}) comprises, together with (a), (b) and, optionally, (d) according to any one of the described examples (for example, (a) chromium picolinate,
(b) phosphatidylcholine or lecithin, (d) rice starch), said at least one first agent (c) comprising or, alternatively, consisting of (c-iii) at least one fatty acid having a number of carbon atoms comprised in the range from C6 to C18, preferably from C12 to C18.

In an alternative example, not encompassed by the wording of the claims, of the present description, the chromium formulation of the description (sucrosomial chromium^{®}) comprises, together with (a), (b) and, optionally, (d) according to any one of the described examples (for example, (a) chromium picolinate,
(b) phosphatidylcholine or lecithin, (d) rice starch)), said at least one first agent (c) comprising or, alternatively, consisting of (c-iv) at least one chitosan derivative, preferably carboxymethylchitosan.

In a further alternative embodiment of the present invention, the chromium formulation of the invention (sucrosomial chromium^{®}) further comprises, together with (a), (b), (c-i) and, optionally, (d) (for example, (a) chromium picolinate, (b) phosphatidylcholine or lecithin, (c-i) sucrester, (d) rice starch)), at least one or two or three of said first agents selected from the group comprising or, alternatively, consisting of: (c-ii) a cyclodextrin, preferably an α-cyclodextrin; (c-iii) a C6-C18 fatty acid isomer, preferably from C12 to C18; (c-iv) a chitosan derivative, preferably carboxymethylchitosan; and mixtures thereof. In other words, one or more of said (c-ii), (c-iii) and/or (c-iv) are additional to (c-i).

The cyclodextrins (CyD or CD) are natural cyclic oligosaccharides consisting of 6, 7 or 8 monomers of D-(+)glucopyranose joined together by an α, 1-4 glycosidic and closed-loop bond. Alpha-cyclodextrin is a 6-monomer cyclic oligosaccharide of D-(+)glucopyranose. An example of a (c-ii) commercial cyclodextrin that can be used in the context of the present invention is the cyclodextrin Cavamax^{®} W6 Food (CAS 10016-20-3) sold by Wecker Chemie AG.

Advantageously, said (c-iii) medium chain fatty acid with a number of carbon atoms C6-C18, preferably C12-C18, linear or branched, saturated or unsaturated, or mixtures of said fatty acids C6-C18 (in short, "C6-C18 fatty acid"), preferably it is lauric acid (C12:0), myristic acid (C14:0), palmitic acid (C16:0) and/or stearic acid (C18:0). Said first agent (c-iii) can be any isomer of a medium chain fatty acid C6-C18, for example any cis isomer or trans isomer of any fatty acid having from 6 to 18 carbon atoms.

In an example not encompassed by the wording of the claims, said (c-iii) medium chain fatty acid with a number of carbon atoms C6-C18, preferably C12-C18, included in the chromium formulation of the description (sucrosomial chromium^{®}) together with (a), (b) and, optionally, (d) according to any one of the described examples, is not a conjugated fatty acid and/or it is not lipoic acid.

Chitosan is a linear polysaccharide consisting of D-glucosamine and N-acetyl-D-glucosamine, linked by β(1-4) bonds. In the context of the present invention, the expression chitosan derivative is used to indicate, by way of non-limiting example, any compound that can be obtained using chitosan or a derivative thereof or a precursor thereof as starting material and subjecting said starting material to chemical transformations known to the man skilled in the art, so that said chitosan derivative has characteristics such to confer to said (a) chromium salt or complex comprised in the formulation of the invention an increased bioavailability and absorption capacity in the organism.

The following embodiments (FRs) of the solid form formulation of chromium of the invention (sucrosomial chromium^{®}) are comprised in the present invention:
- FRa, comprising or, alternatively, consisting of (a), (b), (c-i) and (c-ii) and, optionally, (d);
- FRb, comprising or, alternatively, consisting of (a), (b), (c-i) and (c-iii) and, optionally, (d);
- FRc, comprising or, alternatively, consisting of (a), (b), (c-i) and (c-iv) and, optionally, (d);
- FRd, comprising or, alternatively, consisting of (a), (b), (c-i), (c-ii) and (c-iii) and, optionally, (d);
- FRe, comprising or, alternatively, consisting of (a), (b), (c-i), (c-ii) and (c-iv) and, optionally, (d);
- FRf, comprising or, alternatively, consisting of (a), (b), (c-i), (c-iii) and (c-iv) and, optionally, (d);
- FRg, comprising or, alternatively, consisting of (a), (b) and (c-i) and, optionally, (d);
   wherein in all said embodiments comprised from FRa to FRg the components called (a), (b), (c-i), (c-ii), (c-iii) and (c-iv) are as defined in the present invention.

In an embodiment of the solid form formulation of chromium of the invention (sucrosomial chromium^{®}), components (a), (b), (c-i) and, optionally, (d) are present in the following amounts in % by weight with respect to the total weight of the chromium formulation (sucrosomial chromium^{®}):
- said (a) chromium salt or chromium complex is comprised in an amount such that the chromium element (chromium cation, for example Cr(III), or chromium metal) is comprised in a range from 1% to 40% (for example, 3%, 5%, 7%, 10%, 12%, 15%, 17%, 20%, 25%, or 30%), preferably from 3% to 20%, more preferably from 5% to 15%, for example about 9±1%;
- said (b) phospholipid, preferably phosphoglyceride, more preferably phosphatidylcholine or lecithin, even more preferably solid lecithin (E322) or solid sunflower lecithin (E322), is comprised in a range from 0.05% to 15% (for example, 0.1%, 0.5%, 1%, 3%, 5%, 7%, 10%, or 12%), preferably from 0.1% to 5%, more preferably from 0.1% to 2%, for example about 0.6±0.5%;
- said (c-i), preferably (c-i) sucrester (E473), is comprised in a range from 1% to 70% (for example, 3%, 5%, 7%, 10%, 12%, 15%, 17%, 20%, 25%, 30%, 40%, 50%, or 60%) with respect to the weight of the preparation, preferably from 5% to 50%, more preferably from 10% to 30%, for example about 17±1%;
- if present, said (d) starch, preferably pre-gelatinised rice starch, is comprised in a range from 1% to 50% (for example, 3%, 5%, 7%, 10%, 12%, 15%, 17%, 20%, 25%, 30%, or 40%), preferably from 2% to 40%, more preferably from 3% to 15%, for example about 8±1%.

In an example of the solid form formulation of copper of the description (sucrosomial copper^{®}), the components (a), (b), (c-i) and, optionally, (d) are present in the following amounts in % by weight with respect to the total weight of the copper formulation (sucrosomial copper^{®}):
- said (a) copper salt or copper complex is comprised in an amount such that the copper element (copper cation, for example Cu(II), or copper metal) is comprised in a range from 1% to 30% (for example, 2%, 4%, 6%, 8%, 10%, 12%, 15%, 17%, 20%, or 25%), preferably from 1% to 10% (for example about 6±1%);
- said (b) phospholipid, preferably phosphatidylcholine or lecithin, more preferably sunflower lecithin (e.g. E322), is comprised in a range from 0.05% to 15% (for example, 0.1 %, 0.5%, 1%, 1.5%, 3%, 6%, 8%, 10%, or 12%), preferably from 0.1% to 5%, more preferably from 0.1% to 2%, for example about 0.6±0.5%;
- said (c-i) sucrester (e.g. E473), is comprised in a range from 1% to 70% (for example, 3%, 5%, 7%, 10%, 12%, 15%, 17%, 20%, 25%, 30%, 40%, 50%, or 60%) with respect to the weight of the preparation, preferably from 10% to 25% (for example about 17±1%);
- if present, said (d) starch, preferably pre-gelatinised rice starch, is comprised in a range from 1% to 65% (for example, 10%, 15%, 20%, 25%, 30%, 35%, 40%, 45%, 50%, 55% or 60%), preferably from 30% to 45% (for example about 37±1%).

In an example of the solid form formulation of a vitamin of the description, the components (a), (b), (c-i) and, optionally, (d) are present in said formulation in the following amounts expressed as % by weight with respect to the total weight of the formulation:
- said (a) vitamin C or vitamin B12 or vitamin E is comprised in a range from 20% to 80% (for example, 25%, 35%, 40%, 45% 50%, 55%, 60%, 65%, 70%, or 75%) preferably from 45% to 60% (for example, about 50-55%);
- said (b) phospholipid, preferably phosphatidylcholine or lecithin, more preferably sunflower lecithin (e.g. E322), is comprised in a range from 0.05% to 10%, (for example, 0.1%, 0.2%, 0.3%, 0.4%, 0.5%, 0.6%, 0.7%, 0.8%, 0.9%, 1%, 2%, 3%, 4%, 5% or 8%), preferably from about 0.1% to 2% (for example about 1.0±0.1%);
- said (c-i) sucrester (e.g. E473) is comprised in a range from 1% to 50% (for example, 3%, 5%, 7%, 10%, 12%, 14%, 16%, 18%, 20%, 25%, 30%, or 40%), preferably from about 10% to 25% (for example from about 15±1 to 20±1%); and
- if present, said (d) starch, preferably pre-gelatinised rice starch, is comprised in a range from 5% to 60%, (for example, 10%, 15%, 20%, 25%, 30%, 35%, 40%, 45%, 50%, or 55%), preferably from about 20% to 40% (for example about 30±0.1 %).

In an example of the solid form formulation of a vitamin of the description, the components (a), (b), (c-i) and, optionally, (d) are present in said formulation in the following amounts expressed as % by weight with respect to the total weight of the formulation:
- said (a) vitamin D3 (commercial) is comprised in a percentage (for example 50%, 60%, 70%, 75%, 80%, 85%, 90% or 95%, preferably from about 80% to 90%) such that the intake of pure vitamin D3 (or vitamin D3 as such) is about 0.10%-0.25% (for example, 0.12%, 0.14%, 0.16%, 0.18%, 0.20%, 0.22%, or 0.24%), preferably about 0.20±1%;
- said (b) phospholipid, preferably phosphatidylcholine or lecithin, more preferably sunflower lecithin (e.g. E322), is comprised in a range from 0.05% to 10%, (for example, 0.1%, 0.2%, 0.3%, 0.4%, 0.5%, 0.6%, 0.7%, 0.8%, 0.9%, 1%, 2%, 3%, 4%, 5% or 8%), preferably from about 0.1% to 2% (for example from about 0.5±0.1 a 1.0±0.1%);
- said (c-i) sucrester (e.g. E473) is comprised in a range from 1% to 50% (for example, 3%, 5%, 7%, 10%, 12%, 14%, 16%, 18%, 20%, 25%, 30%, or 40%), preferably from about 5% to 20% (for example from about 10±1 to 15±1%); and
- if present, said (d) starch, preferably pre-gelatinised rice starch, is comprised in a range from 0.05% to 10%, (for example, 0.1%, 0.2%, 0.3%, 0.4%, 0.5%, 0.6%, 0.7%, 0.8%, 0.9%, 1%, 2%, 3%, 4%, 5% or 8%), preferably from about 0.1% to 2% (for example about 1.0±0.1%).

It is clear that, in the solid form formulation of vitamin D3 according to the present description, the amount of commercial vitamin D3 may vary depending on the amount of pure vitamin D3 (or vitamin D3 as such) comprised in said commercial vitamin D3, so as to have - in the formulation of the description - a percentage by weight of pure vitamin D3 of about 0.10%-0.25%, preferably about 0.20±1%, with respect to the weight of the formulation.

Preferably, in the formulation according to the present invention comprising (a) chromium, (b), (c-i) and, optionally, (d)), the (c-i) sucrester: (b) phospholipid weight ratio, preferably a lecithin, more preferably sunflower lecithin, is comprised from 50:1 to 10:1 (for example 45:1, 40:1, 35:1, 30:1, 25:1, 20:1, or 15:1), preferably from 40:1 to 10:1, more preferably from 30:1 to 15:1 (for example about 17: 1 or 17: 0.6-0.5).

For example, in the solid form formulation of chromium according to the present invention, components (a), (b), (c-i) and, optionally, (d) are comprised in said formulations in the following weight percentage with respect to 100 of the total weight of the formulation:
- said (b) phospholipid, preferably lecithin or sunflower lecithin (E322), is comprised from 0.05% to 15% (for example, 0.1%, 0. 5%, 1%, 2%, 3%, 5%, or 10%), preferably from 0.1 % to 2% (for example about 0.5-1.0%;
- said (c-i) sucrester is comprised from 1% to 50% (for example, 5%, 10%, 15%, 20%, 25%, 30% o 40%), preferably from 10% to 25% (for example 13%, 14%, 15%, 16%, 17%, or 18%); and
- said (a) mineral (chromium in the form of a salt or complex) vary according to their weight;
- said (d) optional varies according to the variation of said percentage of mineral.

Forming an object of the present invention is a composition (in short, chromium composition of the invention), preferably in solid form, comprising or, alternatively, consisting of: the solid form formulation of chromium of the invention (sucrosomial chromium^{®}) comprising or, alternatively, consisting of (a), (b), (c-i) and, optionally, (d) according to any one of the embodiments described in the present invention (for example, (a) chromium picolinate, (b) phosphatidylcholine or lecithin, (c-i) sucrester, (d) rice starch) and, optionally, said composition comprises at least one acceptable pharmaceutical or food grade additive and/or excipient.

Forming an object of the present description is a composition (in short, copper composition of the description), preferably in solid form, comprising or, alternatively, consisting of: the solid form formulation of copper of the description (sucrosomial copper^{®}) (comprising or, alternatively, consisting of (a), (b), (c-i) and, optionally, (d) according to any one of the embodiments described in the present description) and at least one acceptable pharmaceutical or food grade additive and/or excipient.

Forming an object of the present description is a composition (in short, composition of at least one vitamin of the description), preferably in solid form, comprising or, alternatively, consisting of: comprising at least one solid form formulation of a vitamin of the description (sucrosomial vitamin^{®}) (comprising or, alternatively, consisting of (a), (b), (c-i) and, optionally, (d) according to any one of the embodiments described in the present description) and at least one acceptable pharmaceutical or food grade additive and/or excipient.

In a first aspect, said composition of the invention may comprise only the solid form formulation of chromium. In a second aspect, said composition of the description, not encompassed by the wording of the claims, may comprise only the solid form formulation of a vitamin (for example, vitamin B12, C, D3 or E) or, alternatively, two, three, or four solid form formulations of said vitamins (for example, vitamin B12, C, D3 and/or E).

In a third aspect, said composition of the invention may comprise the solid form formulation of chromium and a solid form formulation of a vitamin (for example, vitamin B12, C, D3 or E, preferably vitamin B12).

In a fourth aspect, said composition of the invention may comprise the solid form formulation of chromium and two, three, or four solid form formulations of said vitamins (for example, vitamin B12, C, D3 and/or E). In a fifth aspect, said composition of the description, not encompassed by the wording of the claims, may comprise the solid form formulation of copper and a solid form formulation of a vitamin (for example, vitamin B12, C, D3 or E, preferably vitamin B12).

In a sixth aspect, said composition of the description, not encompassed by the wording of the claims, may comprise the solid form formulation of copper and two, three, or four solid form formulations of said vitamins (for example, vitamin B12, C, D3 and/or E).

Examples of compositions according to the present description comprising sucrosomial chromium and/or sucrosomial copper and/or at least one sucrosomial vitamin are as follows:
chromium + vitamin B12 (preferred example) (according to the present invention); chromium + vitamin B12 + at least one vitamin selected from vitamin C, D3, and mixture thereof (according to the present invention);
chromium + copper (according to the present invention); chromium + copper + vitamin B12 (according to the present invention); chromium + copper + vitamin B12 + at least one vitamin selected from vitamin C, D3, and E (according to the present invention);
copper +vitamin B12 (not according to the present invention); copper + at least one vitamin selected from vitamin C, D3, and E (not according to the present invention);
vitamin C + at least one vitamin selected from vitamin D3, B12, and E (not according to the present invention); preferably vitamin C + at least two vitamins selected from vitamin D3, B12, and E (*e.g.* D3 + B12, D3 + E, B12 + E, D3 + B12 + E) (not according to the present invention);
vitamin D3 + at least one vitamin selected from vitamin C, B12, and E (not according to the present invention); preferably vitamin D3 + at least two vitamins selected from vitamin C, B12, and E (*e.g.* C + B12, C + E, B12 + E) (not according to the present invention);
vitamin B12 + at least one vitamin selected from vitamin C, D3, and E (not according to the present invention); preferably vitamin B12 + at least two vitamins selected from vitamin C, D3, and E (*e.g*. C + D3, C + E, D3 + E) (not according to the present invention);
vitamin E + at least one vitamin selected from vitamin C, D3, and B12 (not according to the present invention); preferably vitamin E + at least two vitamins selected from vitamin C, B12, and D3 (e.g. C + D3, C + B12, D3 + B12) (not according to the present invention).

In the composition according to the present invention wherein at least one mineral (chromium) and at least one vitamin are present, each mineral and each vitamin is formulated with (b), (c-i) and, optionally, (d) according to the present invention preferably independently (i.e., separate and independent sucrosomial formulations for each mineral or vitamin).

Said at least one acceptable pharmaceutical or food grade additive and/or excipient may be selected from all the substances known to the man skilled in the art of pharmaceutical or food preparations, such as preservatives, emulsifiers and/or thickeners, such as for example hydroxymethyl cellulose, sweeteners, dyes such as for example dye E171, natural and artificial flavours, antioxidants, stabilisers, fillers, anti-caking agents, such as for example vegetable magnesium stearate, magnesium salts of fatty acids and silicon dioxide, fillers, such as microcrystalline cellulose, and mixtures thereof.

In a preferred embodiment, the composition of the invention comprises a solid form formulation of chromium of the invention (sucrosomial chromium^{®}) comprising or, alternatively, consisting of (a), (b), (c-i) and, optionally, (d) according to any one of the described embodiments; preferably wherein (a) is chromium picolinate (chromium tripicolinate) and/or chromium nicotinate (chromium polynicotinate or chromium trinicotinate).

Advantageously, the composition of the invention, comprising a chromium formulation of the invention (sucrosomial chromium^{®}) comprising or, alternatively, consisting of (a), (b), (c-i) and, optionally, (d) according to any one of the described embodiments, preferably wherein (a) is chromium picolinate , further comprising at least one or more further active components selected from the group comprising or, alternatively, consisting of:
- (e) at least one vitamin selected from the group of vitamins comprising or, alternatively, consisting of: (e-i) a vitamin of group C, (e-ii) a vitamin of group E, (e-iii) a vitamin of group B, preferably vitamin B6 and/or B9 and/or B12, (e-iv) a vitamin of group D, preferably vitamin D3, and mixtures thereof; preferably (e-i) vitamin C (L-ascorbic acid and/or L-sodium ascorbate);
- (f) at least one organic salt or inorganic salt, preferably selected from the group comprising or, alternatively, consisting of: (f-i) magnesium glycinate, (f-ii) selenium methionine, (f-iii) zinc gluconate and mixtures thereof;
- (g) at least one antioxidant, preferably selected from the group comprising or, alternatively, consisting of: (g-i) N-acetylcysteine (NAC), (g-ii) Coenzyme Q10 (CoQ10), (g-iii) Acetyl-L-carnitine (ALC) and mixtures thereof; and
- (h) folic acid.

Advantageously, if present, each single vitamin (e) and/or salt (f) is present in the composition of the invention at an amount equal to 100% RDA (recommended dietary allowance).

Advantageously, if present, each single antioxidant substance (g) is present in the composition of the invention in an amount equal to 100 mg/day.

In a preferred embodiment of the invention, the compositions of the invention are in solid form, for example in solid form as such or in mouth-soluble solid form (or mouth-dispersible solid form, which dissolve in the oral cavity) or in water-dispersible solid form. Alternatively, the compositions of the invention may be in liquid form, for example in the form of solution, dispersion or suspension of a solid in a liquid, or in semi-solid form, for example in form of creams or gels or soft-gels.

In a preferred embodiment of the invention, the compositions of the invention are formulated for oral administration (in short, per os). Advantageously, the compositions of the invention are in solid form as such or mouth-soluble or water-dispersible for oral administration, such as for example powder, granules, microgranules, flakes, tablets or capsules.

If the compositions of the invention are in tablet form, said tablet may have a weight comprised in the range from 200 mg to 2000 mg, for example a hard tablet from 800 mg to 1000 mg.

Said tablets may be coated or filmed with one or more coating layers or films capable of going past the gastric barrier. Said coating may comprise bee wax or a sugar-based solution.

If the compositions of the invention are in the form of a capsule, said capsule may be of hard gelatine or soft gelatine or soft-gel; preferably a gelatine capsule may have a weight comprised in the range from 100 mg to 1500 mg, more preferably about 800 mg.

The composition of the description, comprising said solid form formulation of chromium of the invention (sucrosomial chromium^{®}) and/or said solid form formulation of copper of the description (sucrosomial copper^{®}) and/or said solid form formulation of a vitamin of the description (sucrosomial vitamin^{®}), according to any one of the embodiments described in the present invention, may be a pharmaceutical composition, a medical device composition, a dietary supplement, a food or novel food or nutraceutical composition.

In the context of the present invention, the expression "medical device" is used in the meaning according to the Italian Legislative Decree n° 46 dated 24 February 1997 or according to the new Medical Device Regulation (EU) 2017/745 (MDR).

Forming an object of the present invention is a solid form formulation of chromium of the invention (sucrosomial chromium^{®}) comprising or, alternatively, consisting of (a), (b), (c-i) and, optionally (d) (according to any one of the described embodiments), and the compositions of the invention comprising said chromium formulation of the invention (according to any one of the described embodiments) for use as medicament, both as a primary medicament and as medicament for supporting other medicaments (adjuvant).

Furthermore, forming an object of the present invention is the chromium formulation of the invention (sucrosomial chromium^{®}) and the compositions of the invention comprising said chromium formulation, according to any one of the described embodiments, for use in a method for the preventive and/or curative and/or adjuvant treatment of (I) a chromium insufficiency or deficiency, and of diseases, symptoms and/or disorders related to said deficiency, or for use for chromium supplementation, in subjects in need.

Furthermore, forming an object of the present invention is the chromium formulation of the invention (sucrosomial chromium^{®}) and the compositions of the invention comprising said chromium formulation, according to any one of the described embodiments, for use in a method for the preventive and/or curative and/or adjuvant treatment of (II) a change in the carbohydrate metabolism and/or diseases or symptoms or disorders related thereto, in subjects in need.

Preferably, said change in the carbohydrate metabolism and/or diseases, symptoms or disorders related thereto are selected from:
(II-i) diabetes, preferably type 2 diabetes mellitus,
(II-ii) hyperglycaemia,
(II-iii) insulin resistance,
(II-iv) high absorption of carbohydrates,
(II-v) deregulation of the blood or plasma glucose level, and/or
(II-vi)metabolic syndrome,
and symptoms and/or disorders related to said diseases from (II-i) to (II-vi); preferably (II-i) diabetes, more preferably type 2 diabetes mellitus.

In addition, forming an object of the present invention is the chromium formulation of the invention (sucrosomial chromium^{®}) and the compositions of the invention comprising said chromium formulation, according to any one of the described embodiments, for use in a method for the preventive and/or curative and/or adjuvant treatment of (III.a) a change in the muscle energy metabolism and/or diseases or symptoms or disorders related thereto, in subjects in need.

Preferably, said (III.a) change in the muscle energy metabolism and/or diseases, symptoms or disorders related to said change are selected from: decrease in muscle mass, decrease in muscle strength and decrease in physical resistance to muscle stress and poor or slow recovery of energy after a physical effort.

In addition, forming an object of the present invention is the chromium formulation of the invention (sucrosomial chromium^{®}) and the compositions of the invention comprising said chromium formulation, according to any one of the described embodiments, for use in a method for the preventive and/or curative and/or adjuvant treatment (both therapeutic and non-therapeutic) (III.b) to increase muscle energy metabolism (both for therapeutic purposes and non-therapeutic purposes) and, therefore, to increase muscle mass, to increase muscle strength, increase physical resistance to muscle stress and reduce time to recover energy after a physical effort in a subject in need.

In the context of the present invention, the term "deregulation of blood glucose level" is used to indicate that the glucose level is lower or higher than the range within which the glucose levels of healthy subjects fall as determined by health care organisations (in short, ranges or normal values). For example," normal "blood glucose values are considered to be those comprised in the range from 60 to 110 mg/dl.

Metabolic syndrome is characterised by a large waist circumference (due to excess abdominal fat), arterial hypertension, impaired fasting plasma glucose or insulin resistance and dyslipidaemia. The causes, complications, diagnosis and treatment are similar to those of obesity.

The term "energy metabolism" is used to indicate the combination of processes that generate ATP and energy in another form for the needs of the cell. Energy metabolism begins at the end of the process for breaking down (catabolism) nutrients (carbohydrates, lipids and proteins) and it is characterised by a series of redox reactions in which a reduced substrate (a carbohydrate, a fatty acid and even an amino acid) is oxidised to water and carbon dioxide.

In order to treat (I) a chromium deficiency, or (II) a change in the carbohydrate metabolism, or (III. a) a change in the muscle energy metabolism or in order to (lll.b) increase muscle energy metabolism (both for therapeutic purposes and non-therapeutic purposes), or symptoms and/or disorders related thereto, the daily oral administration of an effective amount of the composition of the invention known to the man skilled in the art is recommended, preferably comprising or, alternatively, consisting of sucrosomoal chromium^{®} such to provide the administration of an amount of chromium element (chromium cation or chromium metal) comprised in the range of from 10 µg to 250 µg or 500 µg (for example 100 µg, 150 µg, 200 µg, 250 µg, 300 µg, 350 µg, 400 µg, or 450 µg), preferably from 50 µg to 250 µg, more preferably from 150 µg to 250 µg, for example about 200-250 µg.

In addition, the compositions of the invention comprising the chromium formulation according to the present invention (sucrosomial chromium^{®}) and, furthermore, at least one formulation of a vitamin (B12, C, D and/or E) according to the present description (sucrosomial vitamin^{®}) are for use as medicament, in particular they are for use in a method for the preventive and/or curative and/or adjuvant treatment of (I) an insufficiency or deficiency of chromium and of said at least one vitamin and diseases, symptoms and/or disorders related to said deficiencies in subjects in need (for example, (II) a change in the carbohydrate metabolism and/or (iii.a) a change in the muscle energy metabolism), or, alternatively, they are for non-therapeutic use (cosmetic use) for the supplementation of chromium and said at least one vitamin in healthy subjects (use (lll.b)).

Forming an object of the present description is a solid form formulation of copper of the invention (sucrosomial copper^{®}) comprising or, alternatively, consisting of (a), (b), (c-i) and, optionally, (d) (according to any one of the described embodiments), and the compositions of the description comprising said copper formulation of the description (according to any one of the described embodiments) for use as medicament, both as a primary medicament and as medicament for supporting other medicaments (adjuvant).

In particular, said copper formulations according to the present description (sucrosomial copper^{®}) and the compositions thereof are for use in a method for the preventive and/or curative and/or adjuvant treatment of (I) a copper insufficiency or deficiency, and of diseases and/or symptoms related to said deficiency, or for use in the supplementation of copper, in subjects in need.

Diseases and/or symptoms related to said copper deficiency may for example be physical fatigue, anaemia, a decrease in the number of white blood cells, osteoporosis, nerve lesions, tingling and loss of sensitivity to feet and hands, or muscle weakness, connective tissue diseases (group of autoimmune diseases characterised by inflammation of the connective tissue), disorders related to oxidative stress (oxidative stress is a mechanism involved in the pathogenesis of many neurodegenerative diseases such as Alzheimer's, Parkinson's and multiple sclerosis).

In order to treat a copper deficiency (both for therapeutic purposes and for non-therapeutic or cosmetic purposes), and disease or symptoms related to said deficiency, the daily oral administration of a therapeutically effective amount known to the man skilled in the art of the copper formulation of the present description (sucrosomial copper^{®}) or of compositions thereof is recommended, for example an amount of copper element (copper (II) or copper metal) comprised in the range from 0.1 mg to 10 mg (for example, 0.5 mg, 1 mg, 1.5 mg, 2 mg, 2.5 mg, 3 mg, 3.5 mg, 4 mg, 5 mg, 6 mg, 7 mg, 8 mg, or 9 mg) preferably from 1 mg to 4 mg, for example about 2 mg.

In addition, the compositions of the description comprising the copper formulation according to the present description (sucrosomial copper^{®}) and, furthermore, at least one formulation of a vitamin (B12, C, D and/or E) according to the present description (sucrosomial vitamin^{®}) are for use as medicament, in particular they are for use in a method for the preventive and/or curative and/or adjuvant treatment of (I) an insufficiency or deficiency of copper and of said at least one vitamin and diseases and/or symptoms related to said deficiencies in subjects in need or, alternatively, they are for non-therapeutic use (cosmetic use) for the supplementation of copper and said at least one vitamin in healthy subjects.

Forming an object of the present description is the solid form formulation of at least one vitamin of the present description (sucrosomial vitamin^{®}, preferably vitamin B12, C, D3 or E) comprising or, alternatively, consisting of (a), (b), (c-i) and, optionally, (d) (according to any one of the described embodiments) and the compositions of the present description comprising said formulation of at least one vitamin for use as medicament, both as primary medicament and as adjuvant.

In particular, said formulations of at least one vitamin according to the present description (sucrosomial vitamin^{®}, preferably B12, C, D3 or E) and the compositions thereof are for use in a method for the preventive and/or curative and/or adjuvant treatment of an insufficiency or deficiency of said vitamins and of diseases and/or symptoms related to said deficiency, or for use in the supplementation of one of said vitamins, in subjects in need.

Diseases and/or symptoms related to said deficiency of at least one vitamin (preferably, vitamin B12, C, D3 or E) may for example be cognitive disorders or change in the cognitive-emotional sphere, cardiometabolic disorders, change in the immune system, stress related to anxiety and depression, fatigue, chronic fatigue and/or asthenia, infections or inflammation of the mucous membranes or the skin, delayed bone growth, problems related to wound healing, and other diseases or symptoms detailed hereinafter, in subjects in need.

Advantageously, the compositions or formulations comprising vitamin B12 (according to any one of the embodiments described in the present description) are used for preventing, reducing or treating a vitamin B12 deficiency. Diseases or symptoms related to a vitamin B12 deficiency are for example: weakness and physical fatigue, shortness of breath, tingling at the tips, memory loss or cognitive difficulties, difficulty in walking due to balance problems, hallucinations, anaemia, and pallor. Said formulation is referred to as "cyclosome or cyclosomal vitamin B12"

Advantageously, the compositions or formulations comprising vitamin C (according to any one of the embodiments described in the present description) are used for preventing, reducing or treating a vitamin C deficiency. Diseases or symptoms related to vitamin C deficiency are for example: fatigue, depression, connective tissue disorders (for example collagen, gingivitis, petechiae, skin rashes, internal bleeding and delayed wound healing), brittleness of the nails, skin and hair following inflammation or medical treatment, and altered bone growth in children, change in the immune system, oxidative stress, decreased iron absorption. Said formulation is referred to as "cyclosome or cyclosomal vitamin C"

Advantageously, the compositions or formulations comprising vitamin D or D3 (according to any one of the embodiments described in the present description) are used for preventing, reducing or treating a vitamin D deficiency. Diseases or symptoms related to a vitamin D deficiency are for example: rickets in children and osteomalacia in adults (due to an alteration of bone mineralization), osteoporosis, and hyperparathyroidism, changes in the immune system. Said formulation is referred to as "cyclosome or cyclosomal vitamin D".

Advantageously, the compositions or formulations comprising vitamin E (according to any one of the embodiments described in the present description) are used for preventing, reducing or treating a vitamin E deficiency. Diseases or symptoms related to vitamin E deficiency (especially in paediatric subjects) are for example: a lack of reflexes and coordination, difficulty walking, muscle weakness, a form of anaemia in which red blood cells are destroyed (haemolytic anaemia), hair loss, skin diseases, weak immune system, fatigue, difficulty concentrating, vision problems, and loss of muscle tone, oxidative stress. Said formulation is referred to as "cyclosome or cyclosomal vitamin E".

In order to treat a vitamin C deficiency (both for therapeutic purposes and for non-therapeutic or cosmetic purposes), and diseases or symptoms related to said deficiency, the daily oral administration of a therapeutically effective amount known to the man skilled in the art of the vitamin C formulation of the present description (sucrosomial vitamin C^{®}) or of compositions thereof is recommended, for example an amount of pure vitamin C (or vitamin C as such) comprised in the range from 100 mg to 1500 mg (for example 200 mg, 400 mg, 600 mg, 800 mg, 1000 mg, or 1200 mg) preferably from 500 mg to 1000 mg, for example about 1000 mg (to date the maximum daily dose allowed is 1000 mg).

In order to treat a vitamin B12 deficiency (both for therapeutic purposes and for non-therapeutic or cosmetic purposes), and diseases or symptoms related to said deficiency, the daily oral administration of a therapeutically effective amount known to the man skilled in the art of the vitamin B12 formulation of the present description (sucrosomial vitamin B12^{®}) or of compositions thereof is recommended, for example an amount of pure vitamin B12 (or vitamin B12 as such) comprised in the range from 0.5 µg to 1000 µg (for example, 1 µg, 1,5 µg, 2 µg, 2,5 µg, 5 µg, 10 µg, 50 µg, 100 µg, 200 µg, 300 µg, 400 µg, 500 µg, 600 µg, 700 µg, 800 µg, or 900 µg) preferably from 1 µg to 5 µg, for example about 2-2.5 µg (to date the maximum daily dose allowed is 1000 µg).

In order to treat a vitamin E deficiency (both for therapeutic purposes and for non-therapeutic or cosmetic purposes), and diseases or symptoms related to said deficiency, the daily oral administration of a therapeutically effective amount known to the man skilled in the art of the vitamin E formulation of the present description (sucrosomial vitamin E^{®}) or of compositions thereof is recommended, for example an amount of pure vitamin E (or vitamin E as such) comprised in the range from 0.5 mg to 80 mg (for example, 1 mg, 5 mg, 10 mg, 15 mg, 20 mg, 30 mg, 40 mg, 50 mg, 60 mg, or 70 mg) preferably from 5 mg to 15 mg, for example about 10 mg (to date the maximum daily dose allowed is 60 mg).

In order to treat a vitamin D3 deficiency (both for therapeutic purposes and for non-therapeutic or cosmetic purposes), and diseases or symptoms related to said deficiency, the daily oral administration of a therapeutically effective amount known to the man skilled in the art of the vitamin D3 formulation of the present description (sucrosomial vitamin D3^{®}) or of compositions thereof is recommended, for example an amount pure vitamin D3 (or vitamin D3 as such) comprised in the range from 1 µg to 100 µg (for example, 5 µg, 10 µg, 15 µg, 20 µg, 25 µg, 30 µg, 50 µg, 100 µg, 150 µg, 200 µg, 250 µg, 300 µg, 350 µg, 400 µg, or 450 µg) preferably from 10 µg to 100 µg, for example about 25-50 µg.

In the context of the present invention, the term "subject/s" is used to indicate human or animal subjects, preferably mammals (e.g. pets such as dogs, cats, horses, sheep or cattle). Preferably, the compositions of the invention are for use in treatment methods for human subjects.

The compositions of the description or the formulation of chromium of the invention (sucrosomial chromium^{®}) or of copper of the description (sucrosomial copper^{®}) or at least one vitamin of the description (sucrosomial vitamin^{®}) may be administered for a period of time comprised in the range from 3 days to 60 or 90 days.

Forming an object of the present invention is the composition of the invention or of the chromium formulation of the invention (sucrosomial chromium^{®}), or, alternatively, of the composition of the invention comprising the chromium formulation according to the present invention and at least one formulation of a vitamin (e.g. B12, C, D and/or E) according to the present invention (sucrosomial chromium^{®} and at least one sucrosomial vitamin^{®}) for use in a method for the preventive and/or curative treatment of a disease, symptom and/or disorder (I), (II) from (II-i) to (II-vi), (iii.a) and/or (Ill.b), as defined in the context of the present invention.

Furthermore, forming an object of the present invention is the use (non-therapeutic) or cosmetic use of the chromium formulation of the invention (sucrosomial chromium^{®}) and of the compositions of the invention comprising said chromium formulation (for example chromium alone or chromium and at least one vitamin), according to any one of the described embodiments, to (lll.b) increase muscle energy metabolism and, therefore, increase muscle mass, increase muscle strength, increase physical resistance to muscle stress and reduce time to recover energy after a physical effort, in a healthy subject.

Forming an object of the present description is the copper formulation of the present description (sucrosomial copper^{®}) or of compositions thereof (copper alone or copper and at least one vitamin) for use a method for the preventive and/or curative treatment of a disease and/or symptom related to a copper insufficiency or deficiency.

Forming an object of the present description is the use (non-therapeutic) or cosmetic use of the copper formulation of the invention (sucrosomial copper^{®}) or of compositions thereof (sucrosomial copper^{®} alone or sucrosomial copper^{®} and at least one sucrosomial vitamin^{®}) to a healthy subject.

Forming an object of the present description is the at least one vitamin formulation of the present description (sucrosomial vitamin^{®}) or of compositions thereof for use in a method for the preventive and/or curative treatment of a disease and/or symptom related to an insufficiency or deficiency of at least one vitamin.

Forming an object of the present description is the use (non-therapeutic) or cosmetic use of the formulation of at least one vitamin of the description (sucrosomial vitamin^{®}) or of compositions thereof to a healthy subject.

Forming an object of the present description is a process for the preparation of the solid form formulation of chromium of the invention (sucrosomial chromium^{®}) or of the solid form formulation of copper of the description (sucrosomial copper^{®}) or of the solid form formulation of at least one vitamin of the description (sucrosomial vitamin^{®}).

In a first embodiment of the invention, said process (in short, first process) is described in patent document WO 2014/009806 A1 from page 7 line 1 to page 8 line 20, incorporated in the present application for reference.

In a second embodiment of the invention, said process (in short, second process) is described in patent document WO 2014/009806 A1 from page 8 line 22 to page 10 line 21, incorporated in the present application for reference.

According to the present invention, said first process and second process are applied as described in patent document WO 2014/009806 A1 considering that (c-ii), (c-iii) and (c-iv) may be used in addition to (c-i), and that the term (b) lecithin should be interpreted as representing the class of phospholipids.

Thus, in the context of the present invention, the term "sucrosomial chromium^{®}" indicates a chromium formulation that can be obtained by processing a chromium salt or complex (a) together with a phospholipid or a phosphatidylcholine or lecithin (b), a sucrester (c-i) and, optionally, a starch (d) according to said first process or said second process, described above (WO 2014/009806 A1) and/or as described in the present description.

In the context of the present description, the term "sucrosomial copper^{®}" indicates a copper formulation that can be obtained by processing a copper salt or complex (a) with a phospholipid (e.g. lecithin) (b), a sucrester (c-i) and, optionally, a starch (d), according to said first process or said second process, described in WO 2014/009806 A1, or as described in the present description.

In the context of the present description, the term "sucrosomal vitamin^{®}" indicates a formulation of a vitamin that can be obtained by processing a vitamin (a) with a phospholipid (e.g. lecithin) (b), a sucrester (c-i) and, optionally, a starch (d), according to said first process or said second process, described in WO 2014/009806 A1, or as described in the present description.

Said process according to the invention is carried out by mixing the components (a), (b), (c-i) and, optionally, (d) according to the percentages by weight defined in the context of the present invention in order to obtain the formulations according to the description comprising chromium (according to the present invention) or copper (not according to the present invention) or a vitamin (not according to the present invention).

Preferably, said process of the description for the preparation of a formulation according to the description comprising chromium (according to the present invention) or copper (not according to the present invention) or a vitamin (not according to the present invention) (preferably chromium alone), for example according to an embodiment described in WO 2014/009806 A1, comprises the steps of
- step 1: preparing a chromium (chromium salt or complex) (according to the present invention) or copper (copper salt or complex) (not according to the present invention) or a vitamin (not according to the present invention), in the form of powder or granules;
- step 2: mixing said (a) chromium (according to the present invention) or copper (not according to the present invention) or vitamin (not according to the present invention) with (b) a phospholipid, preferably lecithin (for example sunflower lecithin) and with (c-i) a sucrester, to obtain a mixture of step 2 or the formulation of the invention; advantageously said lecithin is a hydrolysed or enzymatically hydrolysed lecithin.
- step 3 (optional): mixing said mixture of step 2 with a gelatinised or pre-gelatinised starch of plant origin (d), preferably pre-gelatinised (for example pre-gelatinised rice starch), to obtain the formulation of the invention.
- step 4 (optional after step 2 or after step 3): sieving, preferably with at least one sieve having a size (or nominal sieve opening) comprised in the range from 150 µm to 1400 µm (for example 180, 212, 250, 300, 355, 425, 500, 600, 710, 850, 1000, or 1180 micron), preferably from 600 µm to 850 µm, for example 710 µm (25 US Mesh).

Advantageously, said mixing steps 2 and/or 3 are carried out at room temperature (from 15°C to 30°C, preferably at about 25°C) for a period of time comprised from 10 minutes to 120 minutes, preferably from 15 minutes to 60 minutes, for example about 30 minutes.

Advantageously, said mixing steps 2 and/or 3 are carried out in the absence of solvent (dry mixing).

Said step 2 of mixing the mineral or vitamin with a phospholipid and a sucrester may be divided into two sub-steps in which the first sub-step provides for mixing the mineral or vitamin with a phospholipid and the second sub-step provides for mixing the mixture obtained from the first sub-step with a sucrester.

According to an example of the process of the description for the preparation of a formulation comprising a chromium (according to the present invention) or copper (not according to the present invention) or a vitamin (not according to the present invention), said process does not comprise a spray-dry step.

Forming an object of the present invention is a further process for the preparation of a composition (in short, composition process of the invention) comprising at least one formulation comprising a mineral according to the present invention (chromium) (cyclosomal mineral) and at least one formulation comprising a vitamin according to the present description (such as vitamin A, of group B (e.g. B12), C, D (e.g. D3) and/or E) (cyclosomal vitamin).

Said composition process of the invention comprises the following steps:
- step (i) for preparing (or producing) a solid form formulation of chromium according to the present invention (sucrosomial chromium^{®});
- step (ii) for preparing (or producing) at least one solid form formulation of a vitamin according to the present description (such as, vitamin A, of group B (e.g. B12), C, D (e.g. D3) and/or E) (cyclosomal vitamin);
- step (iii) for mixing said formulation comprising chromium according to the invention and said at least one formulation comprising a vitamin according to the description and at least one additive and/or excipient (i.e. mixing a sucrosomial chromium^{®}, at least one sucrosomial vitamin^{®} and additives-excipients) to obtain a composition according to the invention comprising chromium and at least one vitamin, each in sucrosomial^{®} form.
- step (iv) (optionally), after step (i) or (ii) o (iii), for sieving, preferably with at least one sieve having a size (or nominal sieve opening) comprised in the range from 150 µm to 1400 µm (for example 180 µm, 212 µm, 250 µm, 300 µm, 355 µm, 425 µm, 500 µm, 600 µm, 710 µm, 850 µm, 1000 µm, o 1180 µm), preferably from 600 µm to 850 µm, for example 710 µm (25 US Mesh).

Advantageously, said mixing step (iii) is carried out at room temperature (from 15°C to 30°C, preferably at about 25°C) for a period of time comprised from 10 minutes to 120 minutes, preferably from 15 minutes to 60 minutes, for example about 30 minutes.

A composition that can be obtained according to said composition process of the invention may provide for mixing chromium and a vitamin, or chromium and several vitamins (for example two, three, four, or five vitamins), , wherein preferably chromium and each vitamin are formulated individually in sucrosomial form according to the present invention.

Unless otherwise specified, the term composition or other comprising a component at an amount "comprised in a range from x to y" is used to indicate that said component may be present in the composition or formulation or other at all amounts present in said range, even if not explicitly stated, range extremes comprised.

Unless specified otherwise, the content of a component in a composition refers to the percentage by weight of that component with respect to the total weight of the composition.

Unless specified otherwise, the indication that a composition "comprises" one or more components means that other components - besides the one, or the ones, indicated specifically - can be present and the indication that a composition "consists" of determined components means that the presence of other components is excluded.

Examples of embodiments (FRs- n°) of the present invention are reported below:
FR-1. A solid form formulation of chromium comprising or, alternatively, consisting of:
   (a) a chromium salt or a chromium complex;
   (b) a phospholipid, and
   (c-i) a sucrester or a fatty acid carbohydrate ester.
FR-2. The formulation according to FR-1, wherein said solid form formulation of chromium comprises or, alternatively, consists of:
   (a) a chromium salt or a chromium complex;
   (b) a phospholipid, preferably a phosphoglyceride; and
(c-i) a sucrester or fatty acid carbohydrate ester comprising from 70% by weight to 90% by weight, with respect to the total weight of the sucrester, of monoesters obtained by esterification of sucrose with one or more fatty acids of plant origin, preferably stearic acid and/or palmitic acid; preferably sucrester (E473).
FR-3. The formulation according to FR-1 or FR-2, wherein said (b) phospholipid is a phosphatidylcholine or lecithin; preferably, wherein said (b) phospholipid is a lecithin (E322) selected from sunflower lecithin, corn lecithin, soy lecithin and the mixtures thereof; more preferably wherein said lecithin is a non-hydrolysed lecithin.
FR-4. The formulation according to any one of FR-1 - FR-3, wherein said formulation further comprises (d) a starch of plant origin, preferably gelatinised or pre-gelatinised; more preferably selected from rice starch, soy starch or corn starch; even more preferably wherein said (d) is pre-gelatinised rice starch.
FR-5. The formulation according to any one of FR-1 - FR-4, wherein said (a) at least one chromium salt or chromium complex is selected from the group comprising or, alternatively, consisting of: chromium (iii) picolinate, chromium histidinate, chromium trihistidinate, chromium polyhistidinate, chromium (III) nicotinate, chromium polynicotinate, chromium dinicocysteinate, chromium dinicotinate tryptophan, chromium dinicotinate tyrosine, chromium dinicotinate hydroxycitrate, chromium dinicotinate cinnamate, chromium dinicotinate gallate, chromium dinicotinate 5 hydroxytryptophan, chromium dinicotinate aspartate, chromium dinicotinate glutamate, chromium dinicotinate arginate, chromium tris-tryptophan, chromium nicotinate glycinate, chromium phenylalanine, chromium triphenylalanine, chromium tris-tyrosine, chromium tris-hydroxycitrate, chromium tris-(5-hydroxytryptophan), chromium tris-cinnamate, chromium tris-gallate, chromium acetate, chromium sulphate, chromium chloride, chromium yeast.
FR-6. The formulation according to any one of FR-1 - FR-5, wherein said (a) at least one chromium salt or chromium complex comprises or, alternatively, consists of chromium (III) picolinate.
FR-7. The formulation according to any one of FR-1 - FR-6, wherein said solid form formulation of chromium comprises or, alternatively, consists of:
   (a) chromium (iii) picolinate;
   (b) a phosphatidylcholine or lecithin, preferably sunflower lecithin (E322);
   (c-i) a sucrester or a fatty acid carbohydrate ester,
      preferably a sucrester (E473) comprising from 70% by weight to 90% by weight, with respect to the total weight of the sucrester, of monoesters obtained by esterification of sucrose with one or more fatty acids of plant origin, preferably stearic acid and/or palmitic acid.
   (d) rice starch, preferably pre-gelatinised rice starch.
FR-8. The formulation according to any one of FR-1 - FR-7, wherein a weight ratio (c-i) sucrester : (b) phospholipid is comprised from 50:1 to 10:1, preferably from 40:1 to 10:1, more preferably from 30:1 to 15:1; preferably wherein said (b) phospholipid is a lecithin.
FR-9. A composition comprising or, alternatively, consisting of:
   - the solid form formulation of chromium according to any one of FR-1 - FR-8; and
   - at least one acceptable pharmaceutical or food grade additive and/or excipient.
FR-10. The composition according to FR-9, wherein said composition further comprises a solid form formulation comprising or, alternatively, consisting of:
   (a) a vitamin selected from the group comprising or, alternatively, consisting of: vitamin B12, vitamin C, vitamin D3, and vitamin E;
   (b) a phospholipid,
   (c-i) a sucrester or a fatty acid carbohydrate ester, and, optionally
   (d) a gelatinised or pre-gelatinised starch of plant origin,
FR-11. The composition according to FR-10, wherein said (b) a phospholipid and/or said (c-i) a sucrester and/or said (d) a starch are according to any one of FR- 2 to 8.
FR-12. The formulation or composition according to any one of FR-1 - FR11 for use as medicament.
FR-13. The formulation or composition according to any one of FR-1 - FR-11 for use in a method for the preventive and/or curative treatment of chromium deficiency in a subject in need.
FR-14. The formulation or composition for use according to FR-12 or FR-13, wherein said composition is for use in a method for the preventive and/or curative and/or adjuvant treatment of a change in the carbohydrate metabolism, and symptoms or disorders related thereto, in a subject in need.
FR-15. The formulation or composition for use according to FR-14, wherein said change in the carbohydrate metabolism, or symptom or disorder related thereto, is selected from:
   - diabetes, preferably type 2 diabetes mellitus,
   - hyperglycaemia,
   - insulin resistance,
   - high absorption of carbohydrates,
   - deregulation of the blood or plasma glucose level, and/or
   - metabolic syndrome,
   and symptoms and/or disorders related thereto.
FR-16. The formulation or composition for use according to FR-12 or FR-13, wherein said composition is for use in a method for the preventive and/or curative and/or adjuvant treatment of a change in the muscle energy metabolism, and symptom or disorder related thereto, in a subject in need; preferably, wherein said change in the muscle energy metabolism, or symptom or disorder related thereto is selected from:
   - decrease in muscle mass,
   - decrease in muscle strength,
   - decrease in physical resistance to muscle stress, and/or
   - poor or slow recovery of energy after a physical effort.
FR-17. Non-therapeutic or cosmetic use of the formulation or composition according to any one of FR-1 - FR-11, for increasing the muscle energy metabolism of a healthy subject;
   preferably to increase muscle mass, increase muscle strength, increase physical resistance to muscle stress and/or reducing time to recover energy after a physical effort.
FR-18. A process for the preparation of the solid form formulation of chromium according to any one of FR-1 to 8 comprising the steps of:
   - (1) preparing a chromium salt or complex (a) in the form of powder or granules, to obtain a chromium of step 1;
   - (2) mixing - in the absence of solvent - said chromium of step 1 with a phospholipid (b), preferably lecithin, and a sucrester (c-i) to obtain a mixture or formulation of step 2;
   - (3), optional, mixing said mixture of step 2 with a starch of plant origin (d), preferably gelatinised or pre-gelatinised, more preferably pre-gelatinised rice starch, to obtain the formulation.
FR-19. A process for the preparation of the composition according to FR-10 or FR-11 comprising the steps of:
   - (i) preparing a solid form formulation of chromium according to any one of claims 1 to 8 or producing a solid form formulation of chromium according to claim 18, to obtain said solid form formulation of chromium;
   - (i) preparing at least one solid form formulation of a vitamin according to claim 10 or 11, to obtain said at least one solid form formulation of a vitamin;
   - (iii) mixing said solid form formulation of chromium and said at least one solid form formulation of a vitamin to obtain a mixture of step (iii).
   - (vi) mixing said mixture of step (iii) with at least one additive and/or excipient to obtain the composition.

### EXAMPLES of FORMULATIONS and COMPOSITIONS

### Example 1 (according to the invention):

A solid formulation (about 100 mg) according to the present invention based on chromium (sucrosomial chromium^{®}) consists of: chromium picolinate from about 65 mg to 85 mg (Cr(3+) about 7-12 mg), sunflower lecithin (non-hydrolised) from 0.1 mg to 1 mg, sucrester from 10 mg to 25 mg, pre-gelatinised rice starch from 1 mg to 20 mg.

### Example 2 (according to the invention):

A solid formulation (about 100 mg) according to the present invention based on chromium (sucrosomial chromium^{®}) consists of: chromium picolinate about 74 mg (Cr(3+) about 9 mg), sunflower lecithin (non-hydrolised) about 0.5 mg, sucrester about 17 mg, pre-gelatinised rice starch about 8.5 mg.

### Example 3 (not according to the invention):

A solid formulation (about 30-40 mg) according to the present description based on copper (sucrosomial copperr^{®}) consists of: copper gluconate from about 13.5 mg to 18 mg (Cu(2+) about 2-2.5 mg), lecithin (for example, non-hydrolysed sunflower lecithin) from 0.17 mg to 0.23 mg, sucrester from 5 mg to 7 mg, and pre-gelatinised rice starch from 11 mg to 15 mg.

### Example 4 (according to the invention):

A composition according to the invention comprising sucrosomial chromium^{®} and sucrosomial vitamin B12^{®} comprising:
(A) a solid formulation (about 2.2 mg) according to the present invention based on chromium (sucrosomial chromium^{®}) consists of: chromium picolinate from about 1 mg to 2 mg (for example about 1.65 mg) (equivalent to 0.12-0.24 mg Cr(III); for example 0.20 mg), lecithin (for example, non-hydrolysed sunflower lecithin) from 0.01 mg to 0.02 mg (for example 0.013), (sucrester from 0.3 mg to 0.45 mg (for example about 0.38 mg) (polysaccharide : sucrester weight ratio about 75 : 25 or 50 : 50 or 25 : 75), pre-gelatinised rice starch from 0.1 mg to 0.25 mg (for example about 0.18 mg); and
(B) a solid formulation (5 µg) according to the present description based on vitamin B12 (sucrosomial vitamin B12^{®}) consists of: vitamin B12 from 2 µg to 3 µg (for example about 2.5 µg), lecithin (for example, non-hydrolysed sunflower lecithin) from 0.025 µg to 0.075 µg; sucrester from 0.70 µg to 1 µg, pre-gelatinised rice starch 1.5 µg to 2 µg.

### Example 5 (not according to the invention):

A solid formulation (1-3 g, for example about 2 g) according to the present description based on vitamin C (sucrosomial vitamin C^{®}) consists of: vitamin C from 0.5 g to 2 g (for example about 1 g), lecithin (for example, non-hydrolysed sunflower lecithin) from 0.01 g to 0.03 g, sucrestere from 0.15 g to 0.3 g, and pre-gelatinised rice starch from 0.3 g to 0.6 g.

### Example 6 (not according to the invention)

A solid formulation (2-6 mg, for example about 4 mg) according to the present description based on vitamin B12 (sucrosomial vitamin B12^{®}) consists of: vitamin B12 from 1 mg to 3 mg (for example about 2 mg), lecithin (for example, non-hydrolysed sunflower lecithin) from 0.02 g to 0.06 g, sucrester from 0.3 g to 0.6 g, and pre-gelatinised rice starch from 0.6 g to 1.2 g.

### Example 7 (not according to the invention):

A solid formulation (10-30 mg, for example about 20 mg) according to the present description based on vitamin E (sucrosomial vitamin E^{®}) consists of: vitamin E from 5 mg to 15 mg (for example about 10 mg), lecithin (for example, non-hydrolysed sunflower lecithin) from 0.1 mg to 0.3 mg, sucrester from 1.7 g ma 5 mg, and pre-gelatinised rice starch from 3 mg to 9 mg.

### Example 8 (not according to the invention):

A solid formulation (20-30 mg, for example about 24 mg) according to the present invention based on vitamin D3 (sucrosomial vitamin D3^{®}) consists of: commercial vitamin D3 from about 17 mg to 25 mg (pure vitamin D3 intake from 40 µg to 60 µg (for example about 50 µg), lecithin (for example, non-hydrolysed sunflower lecithin) from 0.14 mg to 0.20 mg (for example about 0.17 mg), sucrester from 2.5 mg to 4 mg (for example about 3 mg), and pre-gelatinised rice starch from 0.1 mg to 0.5 mg.

### EXPERIMENTAL PART

### 1. Study design

*In vitro* efficacy study: evaluation of the modulation of the carbohydrate metabolism by two compositions comprising a chromium salt (Composition 1 and Composition 2) in a culture of differentiated mouse myoblasts.

The following endpoints were considered: - glucose uptake, by means of intracellular glucose assay;
- expression of GLUT-4, insulin-sensitive glucose transporter; and
- ATP content, the primary source of energy for cellular biochemical reactions.

### 2. Tested compositions

Composition 1 (comparative composition): chromium (III) picolinate as such (C₁₈H₁₂N₃O₆Cr, Mw 418,33, CAS 14639-25-9).
Composition 2 (composition according to the invention): sucrosomial chromium^{®}, comprising chromium (iii) picolinate, sunflower lecithin, sucrester and plant starch and having the composition reported in Table 1.

**Table 1**

| Ingredients | Amt. per 100g | Titre Cr element |
|---|---|---|
| Chromium (III) picolinate | 74 | 9% |
| Sunflower lecithin (E322) | 0.6 | |
| Saccharide fatty acid esters (sucrester) | 17.1 | |
| Pre-gelatinised rice starch (*Oryza satira* L.) | 8.3 | |

- Reference compound: metformin hydrochloride.

### 3. Experimental model

The experimental model used in this study is differentiated mouse myoblasts (ATCC Number: CRL-1772, lot 70013341), cultured in Dulbecco's Modified Eagle's Medium (DMEM) supplemented with 10% foetal bovine serum at 37°C, 5% CO₂. The formation of myotubes is induced by adding horse serum.

The experimental protocol provided for:
- Untreated control cells (CTR-);
- Insulin-treated control cells (100 nM);
- Insulin-treated cells and the Compounds in question (Composition 1 and Composition 2);
- Insulin-treated cells and the reference Compound (metformin).

### 4. Experimental treatments

A specific cytotoxicity test on differentiated murine myoblasts was conducted to verify the compatibility of the concentrations to be used in the efficacy test with the cellular system.

Three concentrations of each composition under study (Composition 1 and 2) were tested: 500 µM, 100 µM and 50 µM (Cr). The preparation of the samples involved the use of DMSO as solvent.

The reference Compound (metformin hydrochloride) was tested at 2000 µm.
- The treatment of the cells with Composition 1 and 2 and reference Composition extended for 24 hours. The cultures were then treated with insulin (100 nM) for 4 hours. A source of glucose was added to the media in the glucose uptake evaluation study. The treatments were conducted in quadruplicate.

### 5. Marker assays

At the end of the treatments the cells were lysed to obtain a cell homogenate on which it was assayed:
- glucose, by means of colorimetric assay;
- ATP, by means of colorimetric assay;
- GLUT-4, by means of ELISA technique.

Commercially available colorimetric and ELISA kits were used.

The colorimetric kits use a specific probe, a mixture of reaction enzymes and a curve with known amounts of each standard.

ELISA kits exploit the competitive binding of an antigen (in this case GLUT-4) with its primary antibody. The immune complex (antigen-antibody) is in turn recognised by a secondary antibody conjugated to a peroxidase. The addition of the peroxidase substrate produces a colorimetric reaction with intensity proportional to the amount of immune complexes present and thus to the amount of bound target molecule. Quantitative determination exploits a calibration curve constructed with known and increasing concentrations of standard marker.

### 6. Statistical analysis

The data obtained in the experimental groups treated with the samples subject of the study were subjected to statistical analysis and compared with each other and with insulin according to the t-test. Variations are considered significant for p<0.05.

### 7. Results

### 7.1. GLUCOSE UPTAKE STUDY

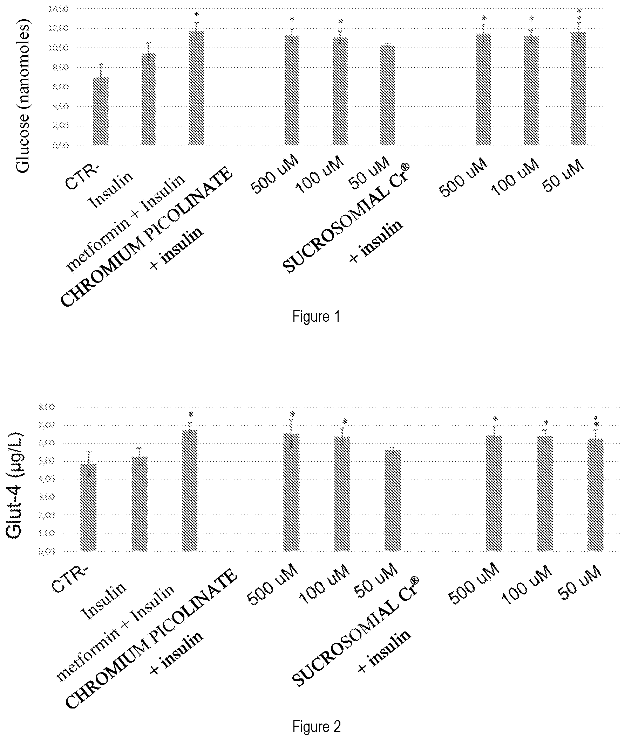

Glucose assay: the results (Table 2 and Figure 1) are expressed as mean content ± st.dev. (expressed in nanomoles) and as a mean % variation with respect to treatment with insulin. Statistically significant data with respect to insulin are reported with *, while ° indicates differences between tested compounds.

Both compositions (Composition 1 and 2) at 500 µM and 100 µM were effective in significantly increasing insulin-dependent glucose uptake, reaching comparable levels of efficacy.

On the other hand, at 50 µM only Composition 2 (sucrosomial chromium^{®}, according to the invention) proved effective in significantly increasing the insulin-dependent glucose uptake. As a matter of fact, Composition 1 (Cr picolinate, reference composition) showed a dose-dependent behaviour, whereas Composition 2 (sucrosomial chromium^{®}, according to the invention) maintained constant levels of efficacy.

**Table 2**

| Treatment | mean value | st.dev | % var. vs. insulin | T-test vs. insulin | Composition T-Test |
|---|---|---|---|---|---|
| CTR- | 6.94 | 1.36 | | | |
| Insulin | 9.42 | 1.10 | - | - | |
| Metformin 2000um + insulin | 11.73 | 0.88 | 24.6% | 0.017 | |

| CR picolinate + insulin | | | | | |
|---|---|---|---|---|---|
| 500 µM | 11.24 | 0.65 | 19.3% | 0.029 | 0.734 |
| 100 µM | 11.08 | 0.62 | 17.6% | 0.039 | 0.795 |
| 50 µM | 10.24 | 0.18 | 8.7% | 0.191 | 0.031 |
| Sucrosomial Cr^{®} + insulin | | | | | |
| 500 µM | 11.45 | 0.98 | 21.6% | 0.033 | |
| 100 µM | 11.20 | 0.59 | 18.8% | 0.029 | |
| 50 µM | 11.64 | 0.98 | 23.6% | 0.024 | |

### 7.2. GLUT-4 STUDY

GLUT-4 assay: the results (Table 3 and Figure 2) are expressed as mean content ± st.dev. (expressed in µg/L) and as a mean % variation with respect to treatment with insulin. Statistically significant data with respect to insulin are reported with *, while ° indicates differences between tested compounds.

Both compositions (Composition 1 and 2) at 500 µM and 100 µM were effective in significantly increasing the levels of GLUT-4 carrier, reaching comparable levels of efficacy.

On the other hand, at 50 µM only Composition 2 (sucrosomial chromium^{®}, according to the invention) proved effective in significantly increasing the levels of GLUT-4 carrier. As a matter of fact, Composition 1 (Cr picolinate, reference composition) showed a dose-dependent behaviour whereas Composition 2 (sucrosomial chromium^{®}, according to the invention) maintained constant levels of efficacy.

**Table 3**

| Treatment | mean value | st.dev | % var. vs. insulin | T-test vs. insulin | Composition T-Test |
|---|---|---|---|---|---|
| CTR- | 4.86 | 0.66 | | | |
| Insulin | 5.24 | 0.48 | - | - | |
| Metformin 2000um + insulin | 6.71 | 0.44 | 28.0% | 0.004 | |

| CR picolinate + insulin | | | | | |
|---|---|---|---|---|---|
| 500 µM | 6.50 | 0.78 | 24.0% | 0.016 | 0.899 |
| 100 µM | 6.35 | 0.62 | 21.1% | 0.039 | 0.945 |
| 50 µM | 5.60 | 0.15 | 6.8% | 0.203 | 0.025 |
| Sucrosomial Cr^{®} + insulin | | | | | |
| 500 µM | 6.44 | 0.50 | 22.9% | 0.014 | |
| 100 µM | 6.37 | 0.34 | 21.5% | 0.009 | |
| 50 µM | 6.28 | 0.43 | 19.7% | 0.018 | |

### 7.3. ATP STUDY

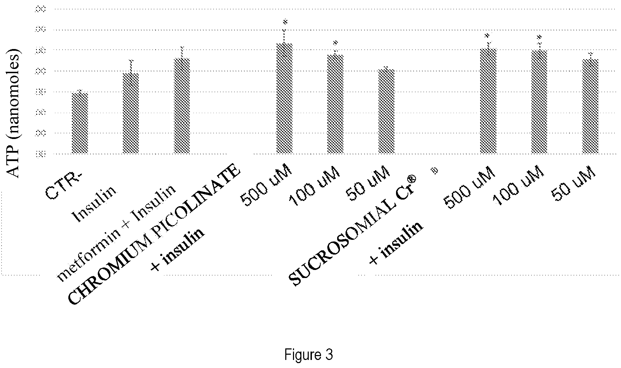

ATP assay: the results (Table 4 and Figure 3) are expressed as mean content ± st.dev. (expressed in nanomoles) and as a mean % variation with respect to treatment with insulin. Statistically significant data with respect to insulin are reported with *, while ° indicates differences between tested compounds.

Both compositions (Composition 1 and 2) at 500 µM and 100 µM were effective in significantly increasing the levels of intracellular ATP, reaching comparable levels of efficacy.

The statistical analysis does not show performance differences between the compositions (Composition 1 and 2) tested at 50 µM, but it can be observed that also in this test Composition 1 (Cr picolinate, reference composition) showed a dose-dependent behaviour, whereas Composition 2 (sucrosomial chromium^{®}, according to the invention) maintained efficacy levels at almost constant levels in all 3 doses tested.

**Table 4**

| Treatment | mean value | st.dev | % var. vs. insulin | T-test vs. insulin | Composition T-Test |
|---|---|---|---|---|---|
| CTR- | 2.93 | 0.14 | | | |
| Insulin | 3.92 | 0.59 | - | - | |
| Metformin 2000um + insulin | 4.61 | 0.53 | 17.6% | 0.133 | |

| CR picolinate + insulin | | | | | |
|---|---|---|---|---|---|
| 500 µM | 5.35 | 0.64 | 36.5% | 0.017 | 0.896 |
| 100 µM | 4.78 | 0.18 | 22.1% | 0.031 | 0.366 |
| 50 µM | 4.09 | 0.26 | 4.3% | 0.620 | 0.054 |

| Sucrosomial Cr^{®} + insulin | | | | | |
|---|---|---|---|---|---|
| 500 µM | 5.08 | 0.29 | 29.7% | 0.013 | |
| 100 µM | 4.98 | 0.37 | 27.1% | 0.023 | |
| 50 µM | 4.56 | 0.30 | 16.4% | 0.102 | |

## Claims

1. A solid form formulation of chromium comprising or, alternatively, consisting of:
(a) a chromium salt or a chromium complex;
(b) a phospholipid, and
(c-i) a sucrester or a fatty acid carbohydrate ester.

2. The formulation according to claim 1, wherein said solid form formulation of chromium comprises or, alternatively, consists of:
(a) a chromium salt or a chromium complex;
(b) a phospholipid, preferably a phosphoglyceride; and
(c-i) a sucrester or fatty acid carbohydrate ester comprising from 70% by weight to 90% by weight, with respect to the total weight of the sucrester, of monoesters obtained by esterification of sucrose with one or more fatty acids of plant origin, preferably stearic acid and/or palmitic acid; preferably sucrester (E473).

3. The formulation according to claim 1 or 2, wherein said (b) phospholipid is a phosphatidylcholine or lecithin; preferably, wherein said (b) phospholipid is a lecithin (E322) selected from sunflower lecithin, corn lecithin, soy lecithin and mixtures thereof; more preferably, wherein said lecithin is a non-hydrolysed lecithin.

4. The formulation according to any one of claims 1-3, wherein said formulation further comprises (d) a starch of plant origin, preferably gelatinised or pre-gelatinised; more preferably selected from rice starch, soy starch or corn starch; even more preferably wherein said (d) is pre-gelatinised rice starch.

5. The formulation according to any one of claims 1- 4, wherein said (a) at least one chromium salt or chromium complex is selected from the group comprising or, alternatively, consisting of: chromium (iii) picolinate, chromium histidinate, chromium trihistidinate, chromium polyhistidinate, chromium (III) nicotinate, chromium polynicotinate, chromium dinicocysteinate, chromium dinicotinate tryptophan, chromium dinicotinate tyrosine, chromium dinicotinate hydroxycitrate, chromium dinicotinate cinnamate, chromium dinicotinate gallate, chromium dinicotinate 5 hydroxytryptophan, chromium dinicotinate aspartate, chromium dinicotinate glutamate, chromium dinicotinate arginate, chromium tris-tryptophan, chromium nicotinate glycinate, chromium phenylalanine, chromium triphenylalanine, chromium tris-tyrosine, chromium tris-hydroxycitrate, chromium tris-(5-hydroxytryptophan), chromium tris-cinnamate, chromium tris-gallate, chromium acetate, chromium sulphate, chromium chloride, chromium yeast.

6. The formulation according to any one of claims 1-5, wherein said (a) at least one chromium salt or chromium complex comprises or, alternatively, consists of chromium (iii) picolinate.

7. The formulation according to any one of claims 1- 6, wherein said solid form formulation of chromium comprises or, alternatively, consists of:
(a) chromium (iii) picolinate;
(b) a phosphatidylcholine or lecithin, preferably sunflower lecithin (E322);
(c-i) a sucrester or a fatty acid carbohydrate ester,
preferably a sucrester (E473) comprising from 70% by weight to 90% by weight, with respect to the total weight of the sucrester, of monoesters obtained by esterification of sucrose with one or more fatty acids of plant origin, preferably stearic acid and/or palmitic acid.
(d) rice starch, preferably pre-gelatinised rice starch.

8. The formulation according to any one of claims 1-7, wherein a weight ratio (c-i) sucrester : (b) phospholipid is comprised from 50:1 to 10:1, preferably from 40:1 to 10:1, more preferably from 30:1 to 15:1;
preferably wherein said (b) phospholipid is a lecithin.

9. A composition comprising or, alternatively, consisting of:
- the solid form formulation of chromium according to any one of claims 1-8; and
- at least one acceptable pharmaceutical or food grade additive and/or excipient.

10. The composition according to claim 9, wherein said composition further comprises a solid form formulation comprising or, alternatively, consisting of:
(a) a vitamin selected from the group comprising or, alternatively, consisting of: vitamin B12, vitamin C, vitamin D3, and vitamin E;
(b) a phospholipid,
(c-i) a sucrester or a fatty acid carbohydrate ester, and, optionally
(d) a gelatinised or pre-gelatinised starch of plant origin,

11. The composition according to claim 10, wherein said (b) a phospholipid and/or said (c-i) a sucrester and/or said (d) a starch are according to any one of claims 2 to 8.

12. The formulation or composition according to any one of the preceding claims for use as medicament.

13. The formulation or composition according to any one of claims 1-11 for use in a method for the preventive and/or curative treatment of chromium deficiency in a subject in need.

14. The formulation or composition for use according to claim 12 or 13, wherein said composition is for use in a method for the preventive and/or curative and/or adjuvant treatment of a change in the carbohydrate metabolism, and symptoms or disorders related thereto, in a subject in need.

15. The formulation or composition for use according to claim 14, wherein said change in the carbohydrate metabolism, or symptom or disorder related thereto, is selected from:
- diabetes, preferably type 2 diabetes mellitus,
- hyperglycaemia,
- insulin resistance,
- high absorption of carbohydrates,
- deregulation of the blood or plasma glucose level, and/or
- metabolic syndrome,
and symptoms and/or disorders related thereto.

16. The formulation or composition for use according to claim 12 or 13, wherein said composition is for use in a method for the preventive and/or curative and/or adjuvant treatment of a change in the muscle energy metabolism, and symptom or disorder related thereto, in a subject in need; preferably, wherein said change in the muscle energy metabolism, or symptom or disorder related thereto is selected from:
- decrease in muscle mass,
- decrease in muscle strength,
- decrease in physical resistance to muscle stress, and/or
- poor or slow recovery of energy after a physical effort.

17. Non-therapeutic or cosmetic use of the formulation or composition according to any one of claims 1-11, for increasing the muscle energy metabolism of a healthy subject;
preferably to increase muscle mass, increase muscle strength, increase physical resistance to muscle stress and/or reducing time to recover energy after a physical effort.

18. A process for the preparation of the solid form formulation of chromium according to any one of claims 1 to 8 comprising the steps of:
- (1) preparing a chromium salt or complex (a) in the form of powder or granules, to obtain a chromium of step 1;
- (2) mixing - in the absence of solvent - said chromium of step 1 with a phospholipid (b), preferably lecithin, and a sucrester (c-i) to obtain a mixture or formulation of step 2;
- (3), optional, mixing said mixture of step 2 with a starch of plant origin (d), preferably gelatinised or pre-gelatinised, more preferably pre-gelatinised rice starch, to obtain the formulation.

19. A process for the preparation of the composition according to claim 10 or 11 comprising the steps of:
- (i) preparing a solid form formulation of chromium according to any one of claims 1 to 8 or producing a solid form formulation of chromium according to claim 18, to obtain said solid form formulation of chromium;
- (i) preparing at least one solid form formulation of a vitamin according to claim 10 or 11, to obtain said at least one solid form formulation of a vitamin;
- (iii) mixing said solid form formulation of chromium and said at least one solid form formulation of a vitamin to obtain a mixture of step (iii).
- (vi) mixing said mixture of step (iii) with at least one additive and/or excipient to obtain the composition.

## Patentansprüche

1. Chromformulierung in fester Form, umfassend oder alternativ bestehend aus:
(a) ein/em Chromsalz oder einen/em Chromkomplex;
(b) ein/em Phospholipid und
(c-i) einen/m Sucrester oder einen/m Fettsäurekohlenhydratester.

2. Formulierung nach Anspruch 1, wobei die Chromformulierung in fester Form umfasst oder alternativ besteht aus:
(a) ein/em Chromsalz oder einen/em Chromkomplex;
(b) ein/em Phospholipid, vorzugsweise ein/em Phosphoglycerid; und
(c-i) einen/m Sucrester oder einen/m Fettsäurekohlenhydratester, der von 70 Gew.- % bis 90 Gew.- %, bezogen auf das Gesamtgewicht des Sucresters, Monoester umfasst, die durch Veresterung von Saccharose mit einer oder mehreren Fettsäuren pflanzlichen Ursprungs, vorzugsweise Stearinsäure und/oder Palmitinsäure erhalten werden; vorzugsweise Sucrester (E473).

3. Formulierung nach Anspruch 1 oder 2, wobei das (b) Phospholipid ein Phosphatidylcholin oder Lecithin ist; vorzugsweise, wobei das (b) Phospholipid ein Lecithin (E322), ausgewählt aus Sonnenblumenlecithin, Maislecithin, Sojalecithin und Mischungen davon, ist; bevorzugter, wobei das Lecithin ein nicht hydrolysiertes Lecithin ist.

4. Formulierung nach einem der Ansprüche 1-3, wobei die Formulierung ferner (d) eine Stärke pflanzlichen Ursprungs, vorzugsweise gelatiniert oder vorgelatiniert, umfasst; bevorzugter ausgewählt aus Reisstärke, Sojastärke oder Maisstärke; noch bevorzugter, wobei (d) vorgelatinierte Reisstärke ist.

5. Formulierung nach einem der Ansprüche 1-4, wobei (a) mindestens ein Chromsalz oder Chromkomplex ausgewählt ist aus der Gruppe umfassend oder alternativ bestehend aus: Chrom (III)-picolinat, Chromhistidinat, Chromtrihistidinat, Chrompolyhistidinat, Chrom (III)-nicotinat, Chrompolynicotinat, Chromdinicocysteinat, Chromdinicotinattryptophan, Chromdinicotinattyrosin, Chromdinicotinathydroxycitrat, Chromdinicotinatcinnamat, Chromdinicotinatgallat, Chromdinicotinat-5-hydroxytryptophan, Chromdinicotinataspartat, Chromdinicotinatglutamat, Chromdinicotinatarginat, Chromtris-tryptophan, Chromnicotinatglycinat, Chromphenylalanin, Chromtriphenylalanin, Chromtristyrosin, Chromtris-hydroxycitrat, Chromtris-(5-hydroxytryptophan), Chromtris-cinnamat, Chromtris-gallat, Chromacetat, Chromsulfat, Chromchlorid, Chromhefe.

6. Formulierung nach einem der Ansprüche 1-5, wobei (a) mindestens ein Chromsalz oder Chromkomplex Chrom (III)-picolinat umfasst oder alternativ daraus besteht.

7. Formulierung nach einem der Ansprüche 1-6, wobei die Chromformulierung in fester Form umfasst oder alternativ besteht aus:
(a) Chrom (III)-picolinat;
(b) ein/em Phosphatidylcholin oder Lecithin, vorzugsweise Sonnenblumenlecithin (E322);
(c-i) einen/m Sucrester oder einen/m Fettsäurekohlenhydratester,
vorzugsweise einen/m Sucrester (E473), der von 70 Gew.- % bis 90 Gew.- %, bezogen auf das Gesamtgewicht des Sucresters, Monoester umfasst, die durch Veresterung von Saccharose mit einer oder mehreren Fettsäuren pflanzlichen Ursprungs, vorzugsweise Stearinsäure und/oder Palmitinsäure, erhalten werden,
(d) Reisstärke, vorzugsweise vorgelatinierte Reisstärke.

8. Formulierung nach einem der Ansprüche 1-7, wobei ein Gewichtsverhältnis (c-i) Sucrester : (b) Phospholipid von 50:1 bis 10:1, vorzugsweise von 40:1 bis 10:1, bevorzugter von 30:1 bis 15:1 enthalten ist;
vorzugsweise wobei das (b) Phospholipid ein Lecithin ist.

9. Zusammensetzung, umfassend oder alternativ bestehend aus:
- die/der Chromformulierung in fester Form nach einem der Ansprüche 1-8; und
- mindestens einen/m akzeptablen pharmazeutischen oder lebensmittelechten Zusatzstoff und/oder Hilfsstoff.

10. Zusammensetzung nach Anspruch 9, wobei die Zusammensetzung ferner eine Formulierung in fester Form umfasst, umfassend oder alternativ bestehend aus:
(a) ein/em Vitamin ausgewählt aus der Gruppe umfassend oder alternativ bestehend aus: Vitamin B12, Vitamin C, Vitamin D3 und Vitamin E;
(b) ein/em Phospholipid,
(c-i) einen/m Sucrester oder einen/m Fettsäurekohlenhydratester und gegebenenfalls
(d) eine/r gelatinierte/n oder vorgelatinierte/n Stärke pflanzlichen Ursprungs.

11. Zusammensetzung nach Anspruch 10, wobei (b) ein Phospholipid und/oder (c-i) ein Sucrester und/oder (d) eine Stärke nach einem der Ansprüche 2 bis 8 sind.

12. Formulierung oder Zusammensetzung nach einem der vorhergehenden Ansprüche zur Verwendung als Arzneimittel.

13. Formulierung oder Zusammensetzung nach einem der Ansprüche 1-11 zur Verwendung in einem Verfahren zur vorbeugenden und/oder kurativen Behandlung von Chrommangel bei einem bedürftigen Patienten.

14. Formulierung oder Zusammensetzung zur Verwendung nach Anspruch 12 oder 13, wobei die Zusammensetzung zur Verwendung in einem Verfahren zur präventiven und/oder kurativen und/oder adjuvanten Behandlung einer Veränderung des Kohlenhydratstoffwechsels und damit zusammenhängender Symptome oder Störungen bei einem bedürftigen Patienten ist.

15. Formulierung oder Zusammensetzung zur Verwendung nach Anspruch 14, wobei die Veränderung des Kohlenhydratstoffwechsels oder ein damit zusammenhängendes Symptom oder eine damit zusammenhängende Störung ausgewählt ist aus:
- Diabetes, vorzugsweise Diabetes mellitus Typ 2,
- Hyperglykämie,
- Insulinresistenz,
- hoher Aufnahme von Kohlenhydraten,
- Deregulierung des Blut- oder Plasmaglukosespiegels und/oder
- metabolischem Syndrom,
und damit zusammenhängenden Symptomen und/oder Störungen.

16. Formulierung oder Zusammensetzung zur Verwendung nach Anspruch 12 oder 13, wobei die Zusammensetzung zur Verwendung in einem Verfahren zur präventiven und/oder kurativen und/oder adjuvanten Behandlung einer Veränderung des Muskel-Energiestoffwechsels und eines damit zusammenhängenden Symptoms oder einer damit zusammenhängenden Störung bei einem bedürftigen Patienten ist; wobei die Veränderung des Muskel-Energiestoffwechsels oder des damit zusammenhängenden Symptoms oder der damit zusammenhängenden Störung vorzugsweise ausgewählt ist aus:
- Abnahme der Muskelmasse,
- Abnahme der Muskelkraft,
- Abnahme der körperlichen Widerstandsfähigkeit gegen Muskelbelastung und/oder
- schlechter oder langsamer Energierückgewinnung nach körperlicher Anstrengung.

17. Nicht-therapeutische oder kosmetische Verwendung der Formulierung oder Zusammensetzung nach einem der Ansprüche 1-11 zur Erhöhung des Muskel-Energiestoffwechsels eines gesunden Probanden; vorzugsweise, um die Muskelmasse zu erhöhen, die Muskelkraft zu erhöhen, die körperliche Widerstandsfähigkeit gegen Muskelbelastung zu erhöhen und/oder die Zeit zu verkürzen, um nach einer körperlichen Anstrengung Energie zurückzugewinnen.

18. Verfahren zur Vorbereitung der Chromformulierung in fester Form nach einem der Ansprüche 1 bis 8, umfassend die Schritte:
- (1) Vorbereiten eines Chromsalzes oder -komplexes (a) in Form von Pulver oder Granulat, um ein Chrom aus Schritt 1 zu erhalten;
- (2) Mischen - in Abwesenheit von Lösungsmittel - des Chroms aus Schritt 1 mit einem Phospholipid (b), vorzugsweise Lecithin, und einem Sucrester (c-i), um eine Mischung oder Formulierung von Schritt 2 zu erhalten;
- (3) optional Mischen der Mischung aus Schritt 2 mit einer Stärke pflanzlichen Ursprungs (d), vorzugsweise gelatinierter oder vorgelatinierter, bevorzugter vorgelatinierter Reisstärke, um die Formulierung zu erhalten.

19. Verfahren zur Vorbereitung der Zusammensetzung nach Anspruch 10 oder 11, umfassend die Schritte:
- (i) Vorbereiten einer Chromformulierung in fester Form nach einem der Ansprüche 1 bis 8 oder Herstellen einer Chromformulierung in fester Form nach Anspruch 18, um die Chromformulierung in fester Form zu erhalten;
- (i) Vorbereiten mindestens einer Formulierung in fester Form eines Vitamins nach Anspruch 10 oder 11, um die mindestens eine Formulierung in fester Form eines Vitamins zu erhalten;
- (iii) Mischen der Chromformulierung in fester Form und der mindestens einen Formulierung in fester Form eines Vitamins, um eine Mischung aus Schritt (iii) zu erhalten;
- (vi) Mischen der Mischung aus Schritt (iii) mit mindestens einem Zusatzstoff und/oder Hilfsstoff, um die Zusammensetzung zu erhalten.

## Revendications

1. Formulation sous forme solide de chrome, comprenant ou, alternativement, étant constituée de :
(a) un sel de chrome ou un complexe de chrome ;
(b) un phospholipide, et
(c-i) un sucroester ou un ester glucidique d'acide gras.

2. Formulation selon la revendication 1, dans laquelle ladite formulation sous forme solide de chrome comprend ou, alternativement, est constituée de :
(a) un sel de chrome ou un complexe de chrome ;
(b) un phospholipide, de préférence un phosphoglycéride ; et
(c-i) un sucroester ou ester glucidique d'acide gras comprenant de 70 % en poids à 90 % en poids, par rapport au poids total de sucroester, de monoesters obtenus par estérification du saccharose avec un ou plusieurs acides gras d'origine végétale, de préférence l'acide stéarique et/ou l'acide palmitique ; de préférence le sucroester (E473).

3. Formulation selon la revendication 1 ou 2, dans laquelle ledit (b) phospholipide est une phosphatidylcholine ou une lécithine ; de préférence, dans laquelle ledit (b) phospholipide est une lécithine (E322) choisie parmi la lécithine de tournesol, la lécithine de maïs, la lécithine de soja et leurs mélanges ; plus préférablement, dans laquelle ladite lécithine est une lécithine non hydrolysée.

4. Formulation selon l'une quelconque des revendications 1-3, dans laquelle ladite formulation comprend de plus (d) un amidon d'origine végétale, de préférence gélatinisé ou prégélatinisé ; plus préférablement choisi parmi l'amidon de riz, l'amidon de soja ou l'amidon de maïs ; plus préférablement encore, dans laquelle ledit (d) est de l'amidon de riz prégélatinisé.

5. Formulation selon l'une quelconque des revendications 1-4, dans laquelle (a) au moins un sel de chrome ou un complexe de chrome est choisi dans le groupe comprenant ou, alternativement, étant constitué de : picolinate de chrome (III), histidinate de chrome, trihistidinate de chrome, polyhistidinate de chrome, nicotinate de chrome (III), polynicotinate de chrome, dinicocystéinate de chrome, tryptophane dinicotinate de chrome, tyrosine dinicotinate de chrome, dinicotinate de chrome hydroxycitrate, dinicotinate de chrome cinnamate, dinicotinate de chrome gallate, dinicotinate de chrome 5 hydroxytryptophane, dinicotinate de chrome aspartate, dinicotinate de chrome glutamate, dinicotinate de chrome arginate, tris-tryptophane de chrome, nicotinate de chrome glycinate, phénylalanine de chrome, triphénylalanine de chrome, tris-tyrosine de chrome, tris-hydroxycitrate de chrome, tris-(5-hydroxytryptophane) de chrome, tris-cinnamate de chrome, tris-gallate de chrome, acétate de chrome, sulfate de chrome, chlorure de chrome, levure de chrome.

6. Formulation selon l'une quelconque des revendications 1-5, dans laquelle ledit (a) au moins un sel de chrome ou un complexe de chrome comprend ou, alternativement, est constitué de picolinate de chrome (III).

7. Formulation selon l'une quelconque des revendications 1-6, dans laquelle ladite formulation sous forme solide de chrome comprend ou, alternativement, est constituée :
(a) de picolinate de chrome (III) ;
(b) d'une phosphatidylcholine ou lécithine, de préférence une lécithine de tournesol (E322) ;
(c-i) un sucroester ou un ester glucidique d'acide gras,
de préférence un sucroester (E473) comprenant de 70 % en poids à 90 % en poids, par rapport au poids total de sucroester, de monoesters obtenus par estérification du saccharose avec un ou plusieurs acides gras d'origine végétale, de préférence l'acide stéarique et/ou l'acide palmitique,
(d) de l'amidon de riz, de préférence de l'amidon de riz prégélatinisé.

8. Formulation selon l'une quelconque des revendications 1-7, dans laquelle le rapport en poids (c-i) de sucroester : (b) phospholipide est compris entre 50:1 et 10:1, de préférence entre 40:1 et 10:1, plus préférablement entre 30:1 et 15:1 ;
de préférence, dans laquelle ledit phospholipide (b) est une lécithine.

9. Composition, comprenant ou, alternativement, étant constituée de :
- la formulation sous forme solide du chrome selon l'une quelconque des revendications 1-8 ; et
- au moins un additif et/ou excipient acceptable de qualité pharmaceutique ou alimentaire.

10. Composition selon la revendication 9, dans laquelle ladite composition comprend de plus une formulation sous forme solide, comprenant ou, alternativement, étant constituée :
(a) d'une vitamine choisie dans le groupe comprenant ou, alternativement, étant constitué de : la vitamine B12, la vitamine C, la vitamine D3, et la vitamine E ;
(b) d'un phospholipide,
(c-i) d'un sucroester ou un ester glucidique d'acide gras, et, éventuellement
(d) d'un amidon gélatinisé ou prégélatinisé d'origine végétale.

11. Composition selon la revendication 10, dans laquelle ledit (b) un phospholipide et/ou ledit (c-i) un sucroester et/ou ledit (d) un amidon sont selon l'une quelconque des revendications 2 à 8.

12. Formulation ou composition selon l'une quelconque des revendications précédentes pour une utilisation en tant que médicament.

13. Formulation ou composition selon l'une quelconque des revendications 1-11 pour une utilisation dans une méthode de traitement préventif et/ou curatif d'une carence en chrome chez un sujet en ayant besoin.

14. Formulation ou composition à utiliser selon la revendication 12 ou 13, dans laquelle ladite composition est à utiliser dans une méthode de traitement préventif et/ou curatif et/ou d'appoint d'une modification du métabolisme des hydrates de carbone, et des symptômes ou troubles qui y sont liés, chez un sujet en ayant besoin.

15. Formulation ou composition à utiliser selon la revendication 14, dans laquelle la modification du métabolisme des hydrates de carbone, ou le symptôme ou le trouble qui y est lié, est choisi parmi :
- les diabètes, de préférence les diabètes sucrés de type 2,
- l'hyperglycémie,
- la résistance à l'insuline,
- l'absorption élevée d'hydrates de carbone,
- la dérégulation du taux de glucose sanguin ou plasmatique, et/ou
- le syndrome métabolique,
ainsi que les symptômes et/ou troubles qui y sont liés.

16. Formulation ou composition à utiliser selon la revendication 12 ou 13, dans laquelle ladite composition est à utiliser dans une méthode de traitement préventif et/ou curatif et/ou d'appoint d'une modification du métabolisme énergétique musculaire, et d'un symptôme ou d'un trouble associé, chez un sujet en ayant besoin ; de préférence, dans laquelle ladite modification du métabolisme énergétique musculaire, ou un symptôme ou un trouble associé, est choisi parmi :
- la diminution de la masse musculaire,
- la diminution de la force musculaire,
- la diminution de la résistance physique au stress musculaire, et/ou
- la récupération faible ou lente de l'énergie après un effort physique.

17. Utilisation non thérapeutique ou cosmétique de la formulation ou de la composition selon l'une quelconque des revendications 1-11, pour augmenter le métabolisme énergétique musculaire d'un sujet sain ;
de préférence pour augmenter la masse musculaire, augmenter la force musculaire, augmenter la résistance physique au stress musculaire et/ou réduire le temps de récupération de l'énergie après un effort physique.

18. Procédé de préparation de la formulation sous forme solide de chrome selon l'une quelconque des revendications 1 à 8, comprenant les étapes de :
- (1) préparer un sel ou complexe de chrome (a) sous forme de poudre ou de granulés, pour obtenir un chrome de l'étape 1 ;
- (2) mélanger - en l'absence de solvant - ledit chrome de l'étape 1 avec un phospholipide (b), de préférence de la lécithine, et un sucroester (c-i) pour obtenir un mélange ou une formulation de l'étape 2 ;
- (3), facultativement, mélanger ledit mélange de l'étape 2 avec un amidon d'origine végétale (d), de préférence gélatinisé ou prégélatinisé, plus préférablement de l'amidon de riz prégélatinisé, pour obtenir la formulation.

19. Procédé de préparation de la composition selon la revendication 10 ou 11, comprenant les étapes de :
- (i) préparer une formulation sous forme solide de chrome selon l'une quelconque des revendications 1 à 8 ou produire une formulation sous forme solide de chrome selon la revendication 18, pour obtenir ladite formulation sous forme solide de chrome ;
- (i) préparer au moins une formulation sous forme solide d'une vitamine selon la revendication 10 ou 11, pour obtenir ladite au moins une formulation sous forme solide d'une vitamine ;
- (iii) mélanger ladite formulation sous forme solide de chrome et ladite au moins une formulation sous forme solide d'une vitamine pour obtenir un mélange de l'étape (iii).
- (vi) mélanger ledit mélange de l'étape (iii) avec au moins un additif et/ou un excipient pour obtenir la composition.
